(19) Europäisches Patentamt
European Patent Office
Office européen des brevets

(11) EP 1 545 512 B1

(12) # EUROPEAN PATENT SPECIFICATION

(45) Date of publication and mention of the grant of the patent:
**20.03.2013 Bulletin 2013/12**

(21) Application number: **03750243.2**

(22) Date of filing: **16.09.2003**

(51) Int Cl.:
***A61K 31/555*** *(2006.01)* ***A61K 31/7072*** *(2006.01)*
***A61P 35/00*** *(2006.01)* ***A61P 31/18*** *(2006.01)*

(86) International application number:
**PCT/CN2003/000780**

(87) International publication number:
**WO 2004/024146 (25.03.2004 Gazette 2004/13)**

(54) **Gold (III) complexes as anti-tumor and anti-HIV agents**

Gold (III)-Komplexe als Antitumor- und Anti-HIV-Mittel

Complexes d'or (III) en tant qu'agents anti-tumoraux et anti-VIH

(84) Designated Contracting States:
**DE GB**

(30) Priority: **16.09.2002 US 411423 P**

(43) Date of publication of application:
**29.06.2005 Bulletin 2005/26**

(73) Proprietor: **The University of Hong Kong**
**Hong Kong (CN)**

(72) Inventor: **CHE, Chiming**
**Hong Kong (CN)**

(74) Representative: **Pluckrose, Anthony William et al**
**Boult Wade Tennant**
**Verulam Gardens**
**70 Gray's Inn Road**
**London WC1X 8BT (GB)**

(56) References cited:
**EP-A- 0 195 147** **US-A- 4 921 847**
**US-A- 5 603 963** **US-A- 5 869 026**
**US-A- 6 087 493**

- **WHEELHOUSE R T ET AL: "CATIONIC PORPHYRINS AS TELOMERASE INHIBITORS: THE INTERACTION OF TETRA-(N-METHYL-4-PYRIDYL)PORPHINE WITH QUADRUPLEX DNA" JOURNAL OF THE AMERICAN CHEMICAL SOCIETY, AMERICAN CHEMICAL SOCIETY, WASHINGTON, DC, US, vol. 120, no. 13, 1998, pages 3261-3262, XP002069116 ISSN: 0002-7863**
- **DONG-FANG SHI ET AL.: "Quadruplex-interactive agents as telomerase inhibitors: synthesis of porphyrins and structure-activity relationship for the inhibition of telomerase" JOURNAL OF MEDICINAL CHEMISTRY, vol. 44, 2001, pages 4509-4523, XP002481451**
- **PASCOLO E ET AL: "Mechanism of human telomerase inhibition by BIBR1532, a synthetic, non-nucleosidic drug candidate", JOURNAL OF BIOLOGICAL CHEMISTRY, AMERICAN SOCIETY FOR BIOCHEMISTRY AND MOLECULAR BIOLOGY, vol. 277, no. 18, 3 May 2002 (2002-05-03), pages 15566-15572, XP008104210, ISSN: 0021-9258, DOI: 10.1074/JBC.M201266200 [retrieved on 2002-02-19]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

**Description**

**Field of the Invention**

The invention is directed to the use of gold(III) complexes as anti-tumor and anti-HIV agents.

**Background of the Invention**

The success of cisplatin as an anti-tumor drug has stimulated considerable interest in using metal complexes as modern therapeutic, diagnostic and radiopharmaceutical agents (*see* Chem. Rev. 99: whole issue (1999)). In view of the emergence of cisplatin-resistant cancer strains and severe toxicity of cisplatin, development of new metal-based drugs with less toxic side effects and ability to overcome the drug resistance are being actively pursued by various research groups worldwide (*see* Clarke et al., Topiccs in Biological Inorganic Chemistry: Metallopharmaceuticals I, DNA Interactions, Springer, Berlin 1999).

Gold compounds have long been used as medicinal agents for rheumatoid arthritis; notable examples include auranofin and myocrysin (*see* Shaw, III, in Gold-Progress in Chemistry, Biochemistry and Technology; Schmidbaur, H., Ed.; Wiley: New York, 1999, 259). The discovery by Lorber and co-workers in 1979 that auranofin could inhibit proliferation of HeLa cells in culture had stimulated an extensive interest in the pharmacological potential of gold compounds (*see* Simon et al., Cancer 44:1965 (1979)), and a series of auranofin derivatives and diphenylphosphinogold(I) complexes was evaluated intensively for *in vitro* and *in vivo* anti-tumor activities during the 1980s (*see* Shaw, III, in Uses of Inorganic Chemistry in Medicine, Farrell, N. P. Ed., 1999, Royal Society of Chemistry: Cambridge, Chapter 3, page 27). Unfortunately, these gold(I) complexes exhibit severe cardiotoxicity in animal studies that rendered them unfavorable for clinical applications. (*See, e.g.,* Hoke et al., Toxicol. Appl. Pharmacol. 100:293 (1989); and Tiekink, Critical Rev. Oncology/Hematology, 42:225-248 (2002)).

Being isostructural and isoelectronic to platinum(II), the Au(III) ion forms square-planar complexes and, therefore, has long been anticipated to be a promising anti-tumor agent. *See* Pieper et al., in Topics in Biological Inorganic Chemistry: Metallopharmaceuticals I, DNA Interactions, Clarke, M. J.; Sadler, P. J. Eds.; Springer: Berlin, 1999; pages 171-199. In contrast to the extensive reports on the biological properties of gold(I) complexes, studies on the anti-tumor and cytotoxic properties of gold(III) complexes are sparse in the literature. The major problem for development of anti-tumor gold(III) compounds is that gold(III) complexes have high redox potential and readily undergo reduction to gold (I) and colloidal gold in physiological buffer solutions.

To date, two major classes of anti-tumor gold(III) complexes (depicted in Scheme 1) have been extensively studied. The class I compounds encompass those gold(III) complexes containing one, two or three labile ligand(s) (e.g., -halide, -SCN) and a mono-/bi-/tridentate auxiliary ligand. For the class II compounds, the four-coordinated gold(III) atom *without labile ligand(s)* is coordinated to some chelating polyamine ligands. According to the literature, all these gold(III) compounds have limited stability in physiological buffer solutions; especially they are readily reduced by mild reductants such as sodium thiosulfate and glutathione to give colloidal gold (see Messori et al., J. Med. Chem. 43:3541 (2000)). To our knowledge, there has been limited success in preparing potent anti-tumor gold(III) compounds that are stable in physiologically relevant conditions. Scheme 1 shows selected literature examples of anti-tumor gold(III) complexes, where X = $I^-$, $Br^-$, $Cl^-$, $SCN^-$ or $CH_3CO_2^-$.

## Scheme 1

**Class I**

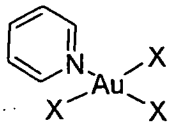

Miraelli, C. K. & co-workers. *Biochem. Pharm.* **1986**, *35*, 1427.

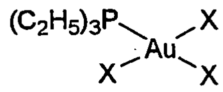

Dabrowiak, J. C. & co-workers. *J. Am. Chem. Soc.* **1987**, *109*, 3810.

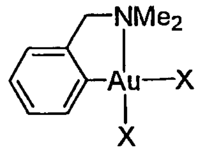

Parish, R. V. & co-workers. *Inorg. Chem.* **1996**, *35*, 1659.

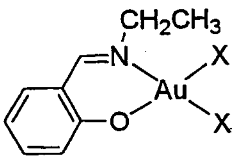

Orioli, P. & co-workers. *Inorg. Chim. Acta* **1999**, *285*, 309.

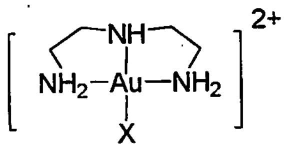

Orioli, P. & co-workers. *Inorg. Chim. Acta* **1998**, *281*, 90.

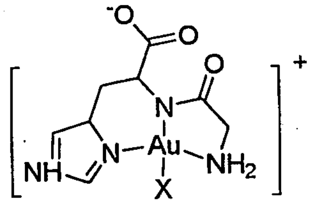

Orioli, P. & co-workers. *Chem.-Biol. Interact.* **2000**, *125*, 29.

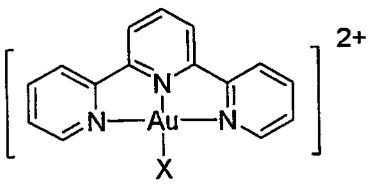

Lippard, S. J. & co-workers. *J. Am. Chem. Soc.* **1983**, *105*, 4293.

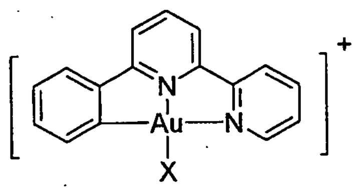

Che. C. -M. & co-workers. *J. Chem. Soc., Chem. Comm.* **1995**, *17*, 1787.

**Class II**

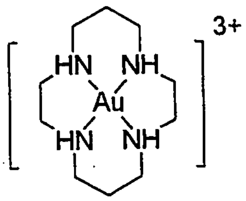

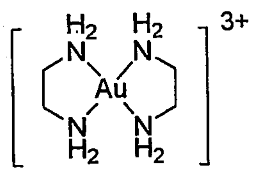

Orioli, P. & co-workers. *J. Med. Chem.* **2000**, *43*, 3541.

Fleischer et al., Inorg. Nucl. Chem. Lett. 5:373 (1969) first reported the synthesis of $[Au^{III}(TPP)]AuCl_4$. *See also* Falk, J. E. Porphyrins and Metalloporphyrins; Elsevier: Amsterdam, 1964. Jamin et al., Inorganica Chimica Acta 27:135 (1978) discloses other gold(III) porphyrin derivatives including $[Au^{III}$(mesoporphyrin IX)$]AuCl_4$; and Abou-Gamra et al., J. Chem. Soc., Faraday Trans. 2, 82:2337 (1986) discloses $[Au^{III}$(*meso*-tetrakis(*N*-methyl-4-pyridyl)porphyrin)$]Cl_5$. These procedures were employed for the preparation of the gold(III) porphyrin complexes reported in this work. Abou-Gamra

et al., J. Chem. Soc., Faraday Trans. 2, 82:2337 (1986) discloses the electrochemistry of gold(III) porphyrins in aqueous media, while Jamin et al., Inorganica Chimica Acta 27:135 (1978) discloses the electrochemistry of gold(III) porphyrins in non-aqueous media. The gold(III) porphyrins were reported to undergo porphyrin-centered redox reactions without reduction of the gold(III) center. However, the biological properties of the gold(III) porphyrin complexes, especially their anti-tumor and cytotoxic properties, are completely unknown in the literature.

Metalloporphyrins are an important class of molecules that form the active sites of numerous proteins for a variety of biological functions including dioxygen transport and storage (hemoglobin, myoglobin), dioxygen activation (cytochrome P-450), electron transport (cytochrome c, cytochrome oxidase) and energy conversion (chlorophyll) (*see* Milgrom, L. R The Colours of Life; Oxford University Press Inc.: New York, 1997; and Kadish, K. M. et al.; The Porphyrin Handbook; Eds.; Academic Press: San Diego, 2000). Pioneered by Fiel and Pasterneck, interaction of metalloporphyrins with DNA has been a subject of intense investigation. Metalloporphyrins have been used as probes for nucleic acid structures and dynamics *(see* Bennett et al., Proc. Natl. Acad. Sci. USA 97:9476 (2000); Guliaev et al., Biochemistry 38:15425 (1999); Lipscomb et al., Biochemistry 35:2818 (1996); and Marzilli et al., J. Am. Chem. Soc. 114:7575 (1992)) and as reagents for DNA footprinting analysis *(see* Mestre et al., Biochemisny 1996, 35:9140 (1996)). Porphyrins and their derivatives have been used in photodynamic cancer therapy; however, few porphyrins and their metal complexes are known to exert significant cytotoxic effects to human cells/ tissues without photoactivation. *See* Hill et al., Proc. Natl. Acad. Sci. USA 92:12126 (1995) and international publication No. WO 00/12512 A1. Recently, Hurley and co-workers found that some cationic *meso*-tetrakis(*N*-methylpyridyl)porphyrins could stabilize G-quadruplex DNA and act as a potential inhibitor of human telomerase for anti-cancer treatment (*see* Han et al, J. Am. Chem. Soc. 121:3561-3570 (1999)). However, there is no evidence that these porphyrins would induce cancer cell death according to Hurley's report.

Human Immunodeficiency Virus type 1 (HIV-1) Reverse Transcriptase (RT) is an important drug target for clinical treatment of Acquired Immunodeficiency Syndrome (AIDS) (*see* Mitsuya et al., Science 249:1533 (1990)). There are two major classes of clinically used HIV-1 reverse transcriptase inhibitors: (1) nucleoside RT inhibitors [NRTIs such as 3-azido-2',3'-dideoxythymidine (AZT), 2',3'-dideoxyinosine (ddI), and 2'-deoxy-3'-thiacytidine (3TC)], and (2) non-nucleoside RT inhibitors (NNRTIs such as nevirapine, delavirdine and efavirenz) (see Anti-AIDS Drug Development: Challenges, Strategies and Prospects (P. Mohan and M. Baba eds. 1995, chapter 11, page 239). NRTIs bind to the normal deoxynucleoside triphosphate (dNTP) substrate-binding site and inhibit HIV replication by terminating DNA chain elongation *(see* Jacobo-Molina et al., Proc. Natl. Acad Sci. USA 90:6320 (1991); and Huang et al., Science 282:1669 (1998)). NNRTIs are believed to inhibit the chemical step of polymerization by binding to a distinct site nearby the polymerase active site. Although the current chemotherapeutic therapy generally involves combinations of both NRTIs and NNRTIs in order to delay emergence of resistance, negative impacts of the treatment including drug toxicity, generation of multidrug-resistant phenotypes and presence of latent virus reservoirs have been reported (*see* Cohen, Science 277:32 (1997)).

Some synthetic metalloporphyrins are reported to inhibit HIV-1 reverse transcriptase activity *in vitro* (see Paterson et al, J. Biol. Chem. 274:1549 (1999); and Staudinger et al., Proc. Assoc. Am. Phys. 108:47 (1996)).

Tetradentate Schiff-base and bis(pyridyl)carboxamide ligands reportedly form complexes with gold(III) ions. *See* Barnholtz et al., Inorg. Chem. 40:972 (2001); Dar et aL, J. Chem. Soc., Dalton Trans. 1907 (1992); Banerjee et al., Ind J. Chem. 23A:555 (1984); Murray et al., J. Organomet. Chem. 61:451 (1973); and Inazu, Bull. Chem. Soc. Jpn. 39: 1065 (1966). Yet, the anti-tumor and cytotoxicities of these complexes remain largely unexplored.

There remains a need, however, for effective anti-cancer agents and anti-HIV agents.

Citation of any reference in this Section of the application is not an admission that the reference is prior art to the application.

US 6,087,493 discloses porphyrin compounds as telomerase inhibitors. Pascolo et al., Journal of Biological Chemistry, American Society for Biochemistry and Molecular Biology, US vol. 277, No. 18, 2002 discloses a Mechanism of human telomerase inhibition by BIBR1532, a synthetic, non-nucleosidic drug candidate.

## Summary of the Invention

The invention relates to gold(III)porphyrin complexes, gold(III)Schiff-base complexes and gold(III)carboxamide complexes ("Gold(III) Complexes") as anti-tumor and anti-HIV agents.

In one embodiment, the invention relates to a composition for use in the treatment of tumor or HIV according to claim 1 of the claims appended hereto.

In another embodiment, the invention relates to a composition for use in the treatment of tumor or HIV according to claim 2 of the claims appended hereto.

In another embodiment, the invention relates to a composition for use in the treatment of tumor or HIV according to claim 7 of the claims appended hereto.

In one embodiment, the invention relates to a pharmaceutical composition according to claim 13 of the claims appended hereto.

In another embodiment, the invention relates to a pharmaceutical composition according to claim 14 of the claims appended hereto.
In another embodiment, the invention relates to a pharmaceutical composition according to claim 15 of the claims appended hereto.

## Brief Description of the Drawings

FIG. 1 shows the UV-visible spectra in dichloromethane of (a) free base $H_2TPP$ and (b) Complex 1a.
FIG. 2 shows the ORTEP drawing of $[Au(TPP)]ClO_4$, the perchlorate salt analog of Complex **1a,** with atom-numbering scheme. Hydrogen atoms and the perchlorate ion are omitted for clarity. Thermal ellipsoids are drawn at the 30% probability level.
FIG. 3 shows the UV-visible spectrum of Complex **1a** (2.5 μM) in Tris buffer/MeCN (19:1).
FIG. 4 shows the UV-visible spectra of Complex **1** (2.5 μ) in 2 mM GSH Tris buffer/ MeCN (19:1) (a) time = 0 h and (b) time = 48 h.
FIG. 5 shows the cyclic voltammogram of Complex **1a** recorded in acetonitrile (0.1 M $Bu_4NPF_6$). Working electrode = glassy carbon, counter-electrode = platinum wire, reference electrode = $Ag/AgNO_3$ (0.1 M in MeCN), scan rate = 200 $mVs^{-1}$.
FIG. 6 shows the linear dual-parameter Hammett correlation (R = 0.99, slope = 1.00) for a series of *para*-substituted Complexes **1c, 1b, 1a, 1d** and **1e**.
FIG. 7 shows the cytoxiticity profiles of (a) human nasopharyngeal carcinoma (SUNE1) and (b) its cisplatin-resistant variant (CNE1) toward Complexes **1a, 1f**, and **1i**. Graphs show the percentage of growth compared to control upon incubation of increasing amounts of the gold(III) complexes. For comparison, curves for $KAu^{III}Cl_4$ and $Pt(NH_3)_2Cl_2$ (cisplatin) are also shown.
FIG. 8 shows the electrophoresis of a 9.4 kbp plasmid (pDR2) in 1% (w/v) agarose gel after restriction enzyme (ApaI) digestion in the absence (lane A) and presence (lane C-H) of various compounds labeled with the [compound]/[bp]. Lane B is the undigested DNA.
FIG. 9 shows the UV-vis spectral changes of Complex 1a in Tris buffer with increasing concentration of ctDNA: (a) r = 0, (b) r = 0.21, (c) r = 0.41, (d) r = 0.62, (e) r = 0.83, (f) r = 1.03, (g) r = 1.24, and (h) r = 1.45 at 292.8 K. Inset: plots of $A_o/A$ *vs* [DNA]/[Complex **1a**] and [ctDNA]/$\Delta\varepsilon_{ap}$ *vs* [ctDNA]. Absorbance was monitored at 410 nm.
FIG. 10 shows the relative viscosity of calf thymus DNA in the presence of ethidium bromide (●), Hoechst 33342 (▲) or Complex **1a** (□), shown as a function of the binding ratio (r).
FIG. 11 shows the gel electrophoresis of 100-bp DNA in 2% (w/v) agarose gel showing the mobilities of the DNA (15.2 μM bp-1) in the absence (lane A & F) and the presence of ethidium bromide (lane B), Hoechst 33342 (lane C), and Complex **1a** (lane D & E, labeled with the [compound]/[bp]).
FIG. 12 shows laser confocal micrographs of the HeLa cells treated with Complex **1a** (0.5 μM ) at time interval of (a) 0 h and (b) 15 h. Apoptotic cells are marked by a circle
FIG. 13 shows the results of apoptotic studies of HeLa cells using flow cytometry: Annexin V/ propidium iodide assay. Flow cytometric results of (a) DMSO, 1.5% (v/v), 15 h; (b) staurosporine, 15 h; (c) Complex **1a**, 6 h and (d) Complex **1a**, 15 h. The corresponding plots of cell counts vs. annexin-V emission are shown in 13(e)-(h).
FIG. 14 shows the results of an HIV-1 RT inhibition study, comparing the activity of Complexes **1j, 1k, 2a** and **3c** with their corresponding free ligands $[H_2TMPyP]^{4+}$, $[H_2TPPS]^{4-}$, $H_2$(Salen) and $H_2(N_4)$. FIG. 14 also includes comparative HIV-1 RT inhibition data for $Au^{III}Cl_4$, $[(Ph_3P)Au^ICl]$, $Zn^{II}$(PP) and $[Zn_{II}(TMPyP)]^{4+}$. The concentration of the metal complexes and free ligands was 6 μM.
FIG. 15 shows the cytotoxicity profiles of the Complexes **1a, 1j, 1k, 2a** and 3c toward human lung fibroblast cell line (CCD-19Lu) as a plot of % cell survival vs log[concentration of gold(III) complex]
FIG. 16 shows the results of an HIV-1 RT inhibition study using Complex **1j** alone and in combination with AZT.

## Detailed Description of the Invention

The present invention is directed to Gold (III) Complexes as anti-tumor and anti-HIV agents.
It will be understood by those skilled in the art that the ligand and the cationic metal center may not form a charge neutral complex. For example, the net positive charge of the cationic metal may be greater than the absolute net negative charge of the deprotonated macrocyle ligand; or the net positive charge of the cationic metal may be less than the absolute net negative charge of the deprotonated macrocyle ligand. In such case, one or more counter-ions will be present to maintain charge neutrality between the ligand and the metal center. Accordingly, the phrase "pharmaceutically acceptable salt," as used herein also includes salts formed from charged metal complex and the counter-ion.
As used herein, the phrase "counter-anion" refers to an anion associated with a positively charged Gold(III) Complex. Examples of counter-anions include fluoride, chloride, bromide, iodide, sulfates and phosphates.

As used herein, the phrase "counter-cation" refers to a cation associated with a negatively charged Gold(III) Complex. Examples of counter-cations include $Na^+$ and $K^+$.

As used herein, the term "TPP" means the dianions of *meso*tetrakis(tetraphenyl)porphyrin.

As used herein, the term "GSH" means glutathione.

"-(6 to 10-membered)aryl means an aromatic ring of 6 to 10 members, including both mono- and bicyclic ring systems. Representative "-(6 to 10-membered)aryls include -phenyl, -naphthyl, and indenyl, which may be substituted or unsubstituted.

"-(5- to 10-membered)heteroaryl" means an aromatic heterocycle ring of 5 to 10 members, including both mono- and bicyclic ring systems, where at least one carbon atom of one or both of the rings is replaced with a heteroatom independently selected from nitrogen, oxygen, and sulfur. One or both of the -(5- to 10-membered)heteroaryl's rings contain at least one carbon atom. Representative -(5- to 10-membered)heteroaryls include -pyridyl, -furyl, -benzofuranyl, -thiophenyl, -benzothiophenyl, -quinolinyl, -pyrrolyl, -indolyl, -oxazolyl, -benzoxazolyl, -imidazolyl, -benzimidazolyl, -thiazolyl, -benzothiazolyl, -isoxazolyl, -pyrazolyl, - isothiazolyl, -pyridazinyl, -pyrimidinyl, -pyrazinyl, -triazinyl, -cinnolinyl, -phthalazinyl, -quinazolinyl and the like. The -(5- to 10-membered)heteroaryls may be substutited or unsubstituted.

"-($C_1$-$C_6$)alkyl" means a saturated straight chain or branched non-cyclic hydrocarbon having from 1 to 6 carbon atoms. Representative saturated straight chain -($C_1$-$C_6$)alkyls include -methyl, -ethyl, -n-propyl, -n-butyl, -n-pentyl, and -n-hexyl. Representative saturated branched -($C_1$-$C_6$)alkyls include -isopropyl, -sec-butyl, -isobutyl, - tert-butyl, -isopentyl, -2-methylbutyl, -3-methylbutyl, -2,2-dimethylbutyl, -2,3-dimethylbutyl, -2-methylpentyl, -3-methylpentyl, -4-methylpentyl and the like.

As used herein, the phrase pharmaceutically acceptable carrier" means approved by a regulatory agency of the Federal or a state government or listed in the U.S. Pharmacopeia or other generally recognized pharmacopoeia for use in animals, mammals, and more particularly in humans. Examples of pharmaceutically acceptable carriers include liquids, such as water and oils, including those of petroleum, animal, vegetable, or synthetic origin. Water is a preferred vehicle when the compound of the invention is administered intravenously. Saline solutions and aqueous dextrose and glycerol solutions can also be employed as liquid vehicles, particularly for injectable solutions.

As used herein, the phrase "patient" refers to an animal. Examples of animals include a cow, monkey, horse, sheep, pig, chicken, turkey, quail, cat, dog, mouse, rat, rabbit, and guinea pig, and more preferably a mammal, and most, preferably a human.

As noted above, the present invention relates to compositions useful for the induction of apoptosis of cancer cells.

Scheme 2 shows illustrative examples of gold(III) porphyrin complexes useful in the present invention, where the counter-ion for Complexes **1a-1j** is chloride, and the counter-cation for Complex **1k** is $Na^+$. It should be noted that complex 1j is a comparative example and does not form part of the present invention.

Scheme 2

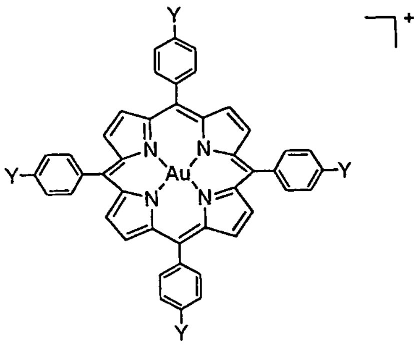

Y = H (**1a**)
= Me (**1b**)
= MeO (**1c**)
= Br (**1d**)
= Cl (**1e**)

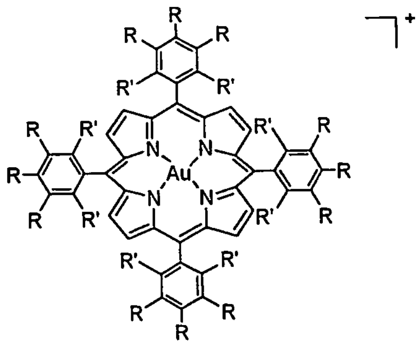

R'= H, R= MeO (**1f**)
R= R'= F (**1g**)

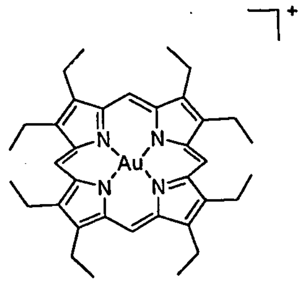

**1h**

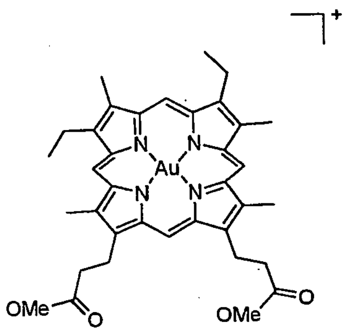

**1i**

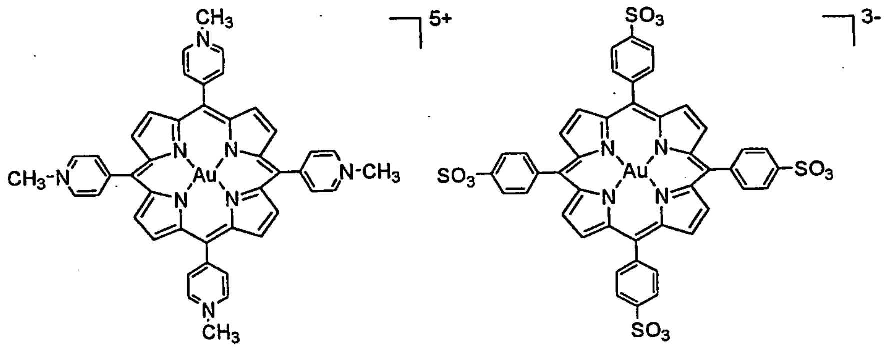

**1j**

**1k**

Scheme 3 shows illustrative examples of gold(III) Shiff base complexes (2) and gold(III) bis(pyridyl)carboxamides complexes (**3**) useful in the present invention, where the counter-anion is $Cl^-$.

Scheme 3

Au$^{III}$(Schiff-base) complexes **2**

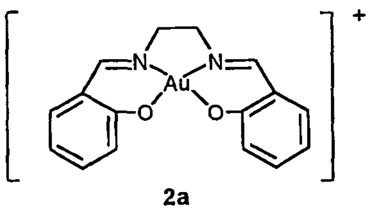

**2a**

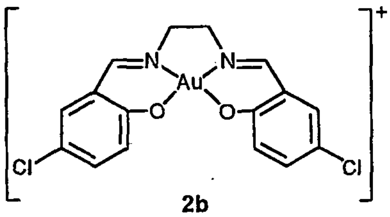

**2b**

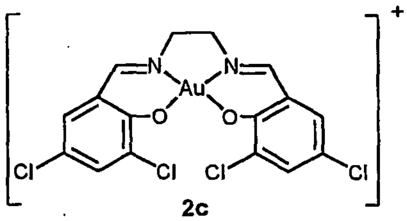

**2c**

Au$^{III}$[bis(pyridyl)carboxamide] complexes **3**

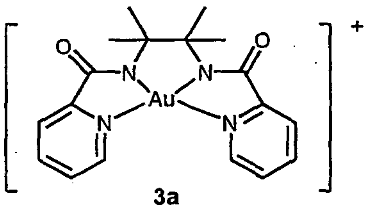

**3a**

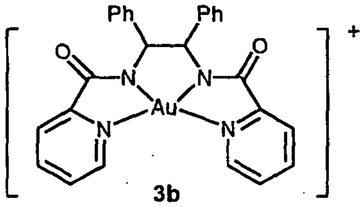

**3b**

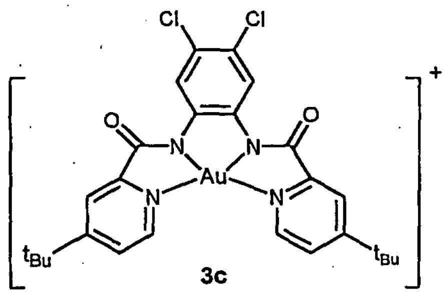

**3c**

The Complexes **1a-i** (Scheme 2) can be prepared according to literature methods with some modifications *(see* MacCragh et al., J. Am. Chem. Soc. 87:2496 (1965) and Fleischer et al., Inorg. Nucl. Chem. Lett. 5:373-376 (1969)) by treating $KAuCl_4$ or $n$$Bu_4NAuCl_4$ with the free base porphyrins in the presence of NaOAc in acetic acid. The reaction are performed under a nitrogen atmosphere using standard Schlenk technique. Complexes **1a-i** (Scheme 2) were This process is depicted in Scheme 4, Route 1.

Scheme 4

***Route 1***: for [Au(III)(tetraarylporphyrins)] (**1a-g**), [Au(III)(OEP)] (**1h**) and [Au(III)(mesoporphyrin IX)] (**1i**)

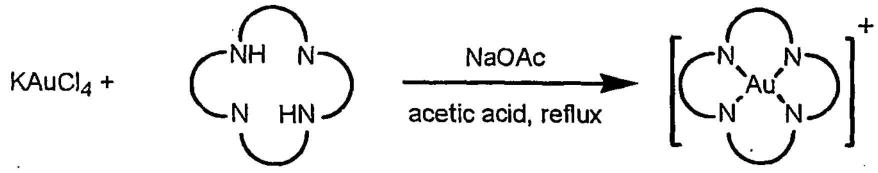


***Route 2***: for [Au(III)(TMPyP]$^{5+}$ (**1j**) and [Au(III)(TPPS)]$^{3-}$ (**1k**)

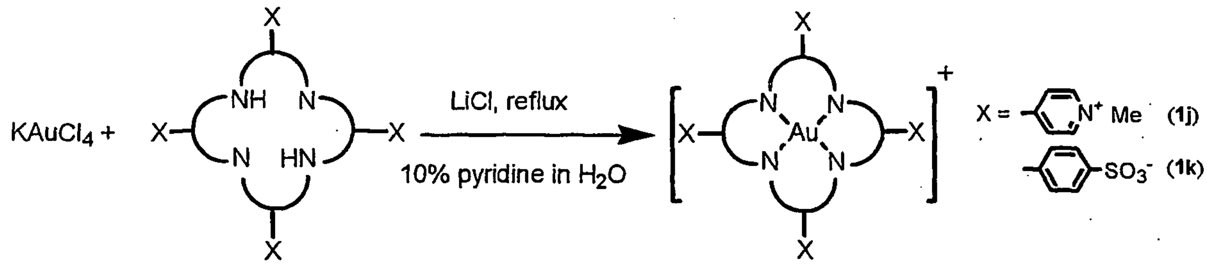


***Route 3***: for [Au(III)(Schiff-base)] complexes (**2a-2c**)

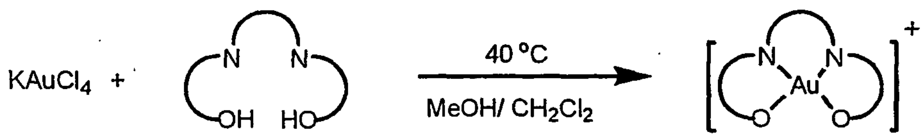


***Route 4***: for [Au(III)(bis-pyridylamide)] complexes (**3a-3c**)

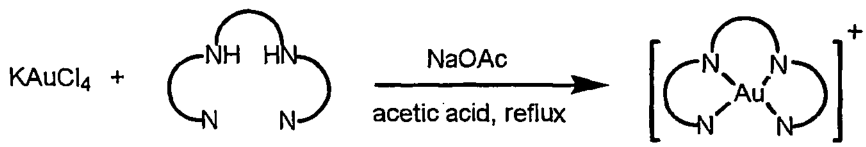


After purification with column chromatography using alumina, followed by metathesis with LiCl in aqueous acetone, analytically pure gold(III) porphyrin complexes were obtained as a chloride salt in 60-70% yields *(see* Examples Section).

Complexes **1j** (reference compound) and **1k** can be prepared according to published procedures in a manner similar to that used to prepare Complexes **1a-1i** except that the reaction was performed using 10% aqueous pyridine as the solvent *(see* Jamin et al., Inorg. Chim. Acta 27:135-143 (1978)); Gibbs et al., J. Inorg. Biochem. 32:39-65 (1988); and Pasternack, J. Inorg. Nucl. Chem. 36:599 (1974)) to afford Complexes **1j** and **1k** in about 35% yield. This procedure is depicted in Scheme 4, Route 2.

The gold(III) Schiff base Complexes **2a-2c** can be prepared in a manner similar to that reported by Barnholtz et al., Inorg. Chem. 40:972 (2001). Treatment of free Schiff-base ligand (0.15 mmol) and $KAuCl_4$ (0.037 mmol) in a $CH_2Cl_2$/MeOH mixture (5:1) at room temperature would afford the gold(III) Schiff-base complexes in about 25% isolated yields. This procedure is depicted in Scheme 4, Rout 4.

The gold(III)bis(pyridyl)carboxamide Complexes **3a-3c** can be prepared in a manner similar to that described above for Complexes **2a-2c** by reaction of $KAuCl_4$ with the free base bis(pyridyl)carboxamides in the presence of NaOAc

in acetic acid. After extensive washing with distilled water and metathesis with LiCl in aqueous acetone, analytically pure gold(III) bis(pyridyl)carboxamide complexes are obtained as chloride salts in 50-60 % yields. This procedures is depicted Scheme 4, Routes 4.

Meso-tetraphenylporphyrin ($H_2TPP$), meso-tetrakis(4-tolyl)porphyrin ($H_2TTP$), meso-tetrakis(4-methoxyphenyl)porphyrin ($H_2TOMePP$), meso-tetrakis(4-bromophenyl)porphyrin ($H_2TBP$), meso-tetrakis(4-chlorophenyl)porphyrin ($H_2TCP$) and meso-tetrakis(3,4,5-trimethoxyphenyl)porphyrin ($H_2TTMPP$) can be prepared according to literature methods or obtained from commercial sources. *See* Adler, J. Org. Chem. 32:476 (1967); Keinan et al., Inorg. Chem. 31: 5433-5438 (1992); and Barnett et al., Tetrahed. Lett. 30:2887-2888 (1973). Meso-tetrakis(N-methylpyridinium-4-yl)porphyrin ($H_2TMPyP$), 2,3,7,8,12,13,17,18-octaethylporphyrin ($H_2OEP$)) and mesoporphyrin IX ($H_2MP$) are available from Aldrich Chemical, Milwaukee, WI. Meso-tetrakis(pentafluorophenyl)porphyrin ($H_2TF5PP$) and meso-tetrakis(4-sulfonatophenyl)porphyrin ($H_2TPPS$) are available from Fluka Chemical (Buchs, Switzerland).

Tetrabutylammonium tetrachloroaurate ($n\text{-}Bu_4NAuCl_4$) can be prepared from the metathesis reaction of [$n$$Bu_4N$]Cl with $HAuCl_4$ in 0.01 M HCl.

In particular, the present invention relates to gold(III) porphyrin complexes containing a tetradentate dianionic macrocyclic ligand (*see* Schemes 2 and 3, class III, for examples) as a class of anti-tumor gold(III) compounds.

Due to high redox potential, gold(III) complexes are usually unstable in solution and readily undergo decomposition to gold(I) / colloidal gold in buffer solution. However, the Gold(III) Complexes of the present invention are stable under physiologically relevant conditions. For example, without being limited by theory, stability of the gold(III) porphyrin complexes **1a-1k** compounds is believed to be due in part to: (1) strong $\sigma$-donors to stabilize oxidizing metal centers, (2) strong chelating effect to avoid undesirable demetallation, and (3) rigid ligand scaffold (especially porphyrin) to stabilize the four-coordinated gold(III) center by raising the kinetic barrier (inner-sphere reorganization energy) for reduction to a two-coordinate gold(I) center.

Examples of porphyrin or porphyrin-like complexes include porphyrins isolated from nature; synthetic porphyrins; phthalocyanines; chlorins; substituted porphyrins or porphyrin-like compounds having symmetrically and unsymmetrically located substituents on any of the positions of the ring periphery; neutrally charged porphyrins; positively charged porphyrins; negatively charged porphyrins; charged porphyrins in combination with counter-ions including alkaline, alkaline earth metal; and rare-earth metal ions.

Examples of antiviral compounds include HPA-23, interferons, ribavirin, phosphonoformate, ansamycin, suramin, imuthiol, pencillamine, carbovir, 3'-azido-3'-deoxythymidine, 2',3'-dideoxycytidine, 2',3'-dideoxyinosine, 2',3'-dideoxyadenosine, 3'-azido-2',3'-dideoxyuridine, 2',3'-dideoxy-2',3'-didehydrocytidine, 3'-deoxy-2',3'-didehydrothymidine and 3'-azido-5-ethyl-2',3'-dideoxyuridine.

Examples of anticancer compounds cisplatin, carboplatin, H E bleomycin, vincristine, vinblastine, doxorubicin, cyclophosphamide, prednisone, methotrexate, dexamethasone, leucovorin and blenoxane.

Examples of cancer cells include mammalian carcinoma cells; and cultured cancer such as Human Cervix Epitheloid Carcinoma cells, Oral Epidermoid carcinoma cells, Nasopharyngeal carcinoma cells, Human Promyelocytic Leukemia cells and Human Hepatocellular Carcinoma cells.

The Gold(III) Complexes are useful for the induction of apoptosis of cancer cells. Cell deaths can be classified into two types, necrosis ("accidental" cell death) and apoptosis ("programmed" cell death). Necrosis causes severe inflammation, but apoptosis does not. Harmlessly disposing of cells (e.g. cancer cells) is one of the considerations in chemotherapy. Therefore, induction of apoptosis is one of the considerations in anti-cancer drug development. Most of the cytotoxic anti-cancer drugs in current clinical uses have been shown to induce apoptosis in susceptible cells. Applicants *in vitro* studies show that that the Gold(III) Complexes are useful for the induction of apoptosis of cancer cells. Without being limited by theory, it is believed that the Gold(III) Complexes "nick" cellular DNA and cause oxidative stress.

In particular, DNA is one of the major targets for anticancer drugs. See Hurley et al., Nature Reviews Cancer, 2:188-200 (2002). It is well known that porphyrins and metalloporphyrins would interact with DNA by intercalation and minor groove binding. Earlier works by Liu and Lippard showed that gold(III) complexes containing polypyridine ligands such as (C^N^N) and terpyridine (terpy) bind to DNA by intercalation *See* Liu et al., J. Chem. Soc., Chem. Comm. 17: 1787-1788 (1995); and Hollis et al., J. Am. Chem. Soc. 105:4293-4299 (1983)]. According to the work by Ward and Dabrowiak, $Et_3PAuBr_3$ would covalently bind to DNA through N7 of guanine (*see* Ward et al., J. Am. Chem. Soc. 1987, 109:3810-3811 (1987). In conjunction to the observed cytotoxicity of the gold(III) porphyrins, we have investigated the binding interaction of Complex **1a** with DNA.

Reverse transcriptase represents one of the major targets in the development of chemotherapeutic drugs against HIV. The Gold(III) Complexes are also useful inhibition of reverse transcriptase. In one embodiment, the reverse transcriptase is the reverse transcriptase of Human Immunodeficiency virus-1.

In some embodiments, the Gold(III) Complexes of the invention may be used in combination with at least one other Gold(II) Complex or another therapeutic agent. Examples of other therapeutic agents include anti-cancer agents and anti-HIV agents. In one embodiment, the anti-HIV agent is AZT.

The following examples are set forth to assist in understanding the invention

## Examples

*Materials.* All chemicals except specially mentioned were purchased from Sigma-Aldrich Chemical Co.(Tokyo, Japan). Acetonitrile and dichloromethane were freshly distilled from $CaH_2$. Analytical grade organic solvents and double distilled deionized water were used throughout the experiments.

The calf thymus DNA was purchased from Sigma-Aldrich Chemical Co. and was purified by phenol-chloroform extraction. The DNA was dissolved in buffer (5 mM Tris, 5 mM NaCl, pH 7.2). The concentration (per base pair) of calf thymus DNA was determined spectrophotometrically on the basic of $\varepsilon_{260}$, 13,200 $M^{-1}cm^{-1}bp$.

Human promyelocytic leukemia (HL-60), human hepatocellular carcinoma (HepG2), Human cervix epitheloid carcinoma cells (HeLa) and human oral epidermoid carcinoma cells (KB-3-1), were from American Type Culture Collection (ATCC) (Rockville, MD, U.S.A.). The multi-drug-resistant variant KB-V1 cell line was obtained by stepwise selections for vinblastine resistance as described by Shen et al., J. Biol. Chem. 261:7762-7770 (1986)], and was generously provided by Dr Michael Gottesman (NIH, Bethesda, MD). Human nasopharyngeal carcinoma cells (SUNE1 and its cisplatin resistance variant, CNE1) were derived from poorly differentiated Nasopharyngeal Carcinoma (NPC) in Chinese patients as described by Gu et al., Chin. J. Cancer 2:270- 272 (1983), and were generously provided by Dr S. W. Tsao (The University of Hong Kong, Hong Kong, PRC). Cell culture flasks and 96 well microtitre plates were from Nalge Nunc Int. Culture medium, other medium constituents and phosphate buffered saline (PBS) were obtained from Gibco BRL (Rock-ville, Maryland, U.S.A.). Cell Proliferation Kit I (MTT) was purchased from Roche (Mannheim, Germany).

*Instrumentation.* All absorption spectra were recorded on a Perkin-Elmer Lambda 19 or a Varian Cary 50 UV-visible spectrophotometer equipped with a PCB-150 water circulator. $^1$H NMR spectra were recorded on Bruker DPX-300 or DPX-500 NMR spectrometers. Positive ion FAB mass spectra were recorded on a Finnigan MAT95 mass spectrometer. Viscosity evaluation was carried out by using a Cannon-Manning Semi-Micro Viscometer (Cannon-Instrument Co., State College, PA). Cyclic voltammetry was performed by using a PAAR model 175 universal programmer and a Model 173 potentiostat, and the cyclic voltammograms were recorded with a Kipp & Zonen BD 90 X-Y recorder at scan rates of 200 $mVs^{-1}$. Flow cytometric analysis was performed with a Coulter EPICS flow cytometer (Coulter, Miami, FL) equipped with 480 long, 525 band and 625 long pass mirrors. Samples were excited by 15 mW air-cool argon convergent laser at 488 nm. Fluorescence signals were manipulated with Coulter Elite 4.0 software (Coulter) and were analyzed by Winlist 1.04 and Modfit 5.11 software (Verity Software House, Topsham, ME).

All the complexes were spectroscopically characterized by UV-vis, $^1$H NMR and FAB-MS with purities of >99% being confirmed by elemental analysis.

## Example 1: Preparation of the Gold(III) Complexes

Example 1 describe the preparation and characterization of illustrative Gold(III) Complexes **1a-1k**.

*Gold(III) Complexes **1a-1i**.* The synthesis of the gold(III) porphyrins was performed under a nitrogen atmosphere using the standard Schlenk technique. Complexes **1a-i** (Scheme 2) were prepared according to a literature method with some modifications. *See* MacCragh et al., J. Am. Chem. Soc. 87:2496 (1965) and Fleischer et al., Inorg. Nucl. Chem. Lett. 5:373-376 (1969). In general, $KAuCl_4$ or [*n*$Bu_4$N]$AuCl_4$ (0.508 mmol) and sodium acetate (2.538 mmol) were heated to 80 °C in acetic acid (20 ml) for 15 minutes. A solution of free porphyrin (0.406 mmol) in acetic acid (10 ml) was added dropwise. The mixture was heated under reflux for 0.5 to 2 h, and the completion of metallation was checked by disappearance of the Q band using UV-Vis spectrophotometry. Upon removal of solvent under vacuum, the residue was dissolved in $CH_2Cl_2$ (40 ml). The $CH_2Cl_2$ solution was washed twice with water (2 x 40 ml) to remove any unreacted $KAuCl_4$ and NaOAc, and pre-concentrated down to 3 ml. It was chromatographed on a neutral 90-alumina packed column. The column was eluted with $CH_2Cl_2$ to remove the unreacted free base porphyrin, and the gold(III) porphyrin complex was eluted using $CH_2Cl_2$/ MeOH (99:1, v/v). A reddish-purple solid was obtained after solvent evaporation and the complex was recrystallized from a $CHCl_3$/ petroleum ether (1:1, v/v) mixture.

*Gold(III) Complexes **1j-k**.* Complexes **1j** (reference compound) and **1k** were prepared according to published procedures. See Gibbs et al., J. Inorg. Biochem. 32:39-65 (1988); and Pasternack, J. Inorg. Nucl. Chem., 36:599 (1974).

Gold(III) Complexes **1a-1k** exhibited well-resolved $^1$H NMR spectra. For complexes **1a-1g** and **1j-1k**, all the pyrrolic hydrogen atoms are equivalent and are consistent with a $D_4$ symmetry.

Analytical data for Complexes 1a-1k are shown below:

Complex **1a**: UV-vis ($CH_2Cl_2$) $\lambda_{max}$/nm (log ε): 409 (5.68), 521 (4.73). $^1$H NMR ($CDCl_3$): δ 9.28 (s, 8H), 7.89 (m, 8H), 8.24 (d, 8H) 7.89 (m, 4H). m/z = 809. Yield 70%. Anal. Calcd. for $C_{44}H_{28}N_4ClAu$ (%): C, 62.53; H, 3.34; N, 6.63. Found: C, 62.50; H, 3.61; N, 6.74.

Complex **1b:** UV-vis ($CH_2Cl_2$) $\lambda_{max}$/nm (log ε): 413 (5.41), 522 (4.62). $^1$H NMR ($CDCl_3$): δ 9.29 (s, 8H), 7.67 (d, 8H), 8.10 (d, 8H) 2.75 (s, 12H). m/z = 865. Yield 69%. Anal. Calcd. for $C_{48}H_{36}N_4ClAu$ (%): C, 63.97; H, 4.03; N, 6.22. Found: C, 63.82; H, 4.39; N, 5.82.

Complex **1c**: UV-vis ($CH_2Cl_2$) $\lambda_{max}$/nm (log ε): 423 (5.26), 527 (4.34). $^1$H NMR ($CDCl_3$): δ 9.31 (s, 8H), 7.38 (d,

8H), 8.13 (d, 8H) 4.13 (s, 12H). m/z = 929. Yield 63%. Anal. Calcd for $C_{48}H_{36}N_4O_4ClAu$ (%): C, 59.73; H, 3.76; N, 5.80. Found: C, 59.55; H, 3.49; N, 5.99.

Complex **1d**: UV-vis ($CH_2Cl_2$) $\lambda_{max}$/nm (log ε): 411 (5.53), 522 (4.48). $^1$H NMR ($CDCl_3$): δ 9.24 (s, 8H), 7.80 (d, 8H), 8.14 (d, 8H). m/z = 1125. Yield 60%. Anal. Calcd. for $C_{44}H_{24}N_4Br_4ClAu$ (%): C, 45.53; H, 2.08; N, 4.83. Found: C, 46.01; H, 2.35; N, 4.79.

Complex **1e**: UV-vis ($CH_2Cl_2$) $\lambda_{max}$/nm (log ε): 409 (5.47), 520 (4.34). $^1$H NMR ($CDCl_3$): δ 9.23 (s, 8H), 7.88 (d, 8H), 8.20 (d, 8H). m/z = 947. Yield 61%. Anal. Calcd. for $C_{44}H_{24}N_4Cl_5Au$ (%): C, 53.77; H, 2.46; N, 5.70. Found: C, 53.46; H, 2.72 N, 5.68.

Complex **1f**: UV-vis ($CH_2Cl_2$) $\lambda_{max}$/nm (log ε): 423 (5.18), 526 (4.17). $^1$H NMR ($CDCl_3$): δ 9.36 (s, 8H), 7.57 (s, 8H), 3.98 (s, 24H) 4.19 (s, 12H). m/z = 1169. Yield 70%. Anal. Calcd. for $C_{56}H_{52}O_{12}N_4ClAu$ (%): C, 55.80; H, 4.35; N, 4.65. Found: C, 55.56; H, 4.28; N, 4.57.

Complex **1g**: UV-vis ($CH_2Cl_2$) $\lambda_{max}$/nm (log ε): 402 (5.71), 518 (4.55), 552 (4.10). $^1$H NMR ($CDCl_3$): δ 9.48 (s, 8H). m/z = 1170. Yield 60%. Anal. Calcd. for $C_{44}H_8N_4F_{20}ClAu$: C, 43.86; H, 0.67; N, 4.65. Found: C, 43.93; H, 0.65; N, 4.62.

Complex **1h**: UV-vis ($CH_2Cl_2$) $\lambda_{max}$/mm (log ε): 389 (5.35), 510 (3.98), 545 (4.23). $^1$H-NMR ($CDCl_3$): δ 10.51 (s, 8H), 2.10 (t, 24H), 1.59 (q, 16H) 7.89 (m, 4H). m/z = 731. Yield 62%. Anal. Calcd. for $C_{36}H_{44}N_4ClAu$ (%): C, 56.51; H, 5.80; N, 7.32. Found: C, 56.14; H, 5.67; N, 7.58.

Complex **1i**: UV-vis ($CH_2Cl_2$) $\lambda_{max}$/nm (log ε): 398 (5.12), 518 (3.89), 548 (4.12). $^1$H NMR ($CDCl_3$): δ 10.63 (s, 4H). m/z = 789. Yield 64%. Anal. Calcd. for $C_{36}H_{42}O_4N_4ClAu$ (%): C, 52.27; H, 5.12; N, 6.77. Found: C, 51.94; H, 4.85; N, 6.57.

Complex **1j**: UV-vis ($H_2O$) $\lambda_{max}$/nm (log ε): 405 (4.89), 522 (3.98), 556 (3.64). $^1$H NMR ($D_2O$): δ 9.52 (s, 8H), 9.33 (d, 8H), 9.03 (d, 8H) 2.07 (s, 12H). m/z = 175. Yield 34%. Anal. Calcd. for $C_{44}H_{60}N_8O_{12}Cl_5Au$ (%): C, 41.70; H, 4.77; N, 8.84. Found: C, 41.98; H, 4.65; N, 8.57.

Complex **1k**: UV-vis ($H_2O$) $\lambda_{max}$/nm (log ε): 407 (5.34), 521 (4.39). $^1$H NMR ($D_2O$): δ 8.62 (s, 8H), 7.69 (d, 8H), 8.24 (d, 8H). m/z = 375. Yield 42%. Anal. Calcd. for $C_{44}H_{40}N_4$ $O_{20}ClS_4Na_4Au$ (%): C, 37.82; H, 2.89; N, 4.01. Found: C, 37.53; H, 3.01; N, 4.17.

The UV-vis spectra of Complexes **1a-1k** (the gold porphyrin complexes) feature an intense Soret band and a weaker Q band. Compared to the spectrum of a closed d-shell metalloporphyrin, the Soret bands of the metal complexes of the invention display a characteristic blue-shift in absorption energy *(hypso,* see FIG. 1). Without being bound by theory, it is believed that the Goutermann's four-orbital-model, the *hypso* spectrum for gold(III) porphyrins is the result of metal-to-ligand π-back bonding interaction between the metal filled $d_\pi$ and the vacant π* orbital of the porphyrin ligand.

*X-ray Crystal Determination.* Crystals of Complex **1a** were obtained by a slow diffusion of n-pentane into a solution of Complex **1a** in chloroform. A purple crystal having dimensions 0.4 x 0.1 x 0.1 mm mounted on a glass fiber was used for data collection at 28°C on a MAR diffractometer with a 300 mm image plate detector using graphite monochromatized Mo-$K_\alpha$ radiation (λ = 0.71073 Å). Data collection was made with 3° oscillation step of φ, 300 seconds exposure time and scanner distance at 120 mm. 48 images were collected. The images were interpreted and intensities integrated using program DENZO [Otwinowski, Z. and Minor, W. In Processing of X-ray Diffraction Data Collected in Oscillation Mode, Methods in Enzymology, Carter C. W., Sweet Jr. & R. M., Eds.; Academic Press.: 1997; Vol. 276, p. 307-326].. The structure was solved by direct methods employing SHELXS-97 program [Sheldrick, G., M. SHELX97. Programs for Crystal Structure Analysis (Release 97-2). University of Goetingen, Germany, 1997]. Au, Cl and many non-H atoms were located according to the direct methods. The positions of the other non-hydrogen atoms were found after successful refinement by full-matrix least-squares using program SHELXL-97.

The molecular structure of Complex **1a** was established by X-ray crystallography (FIG. 2). Crystallographic data collection parameters and selected bond angles and distances for Complex **1a** are summarized in Table 1 and Table 2, respectively. As shown in FIG. 2, the gold(III) center is four-coordinated and is located within the plane of the tetrapyrrole macrocycle. The Au-N distances are 2.032(5) and 2.033(5) Å, which are comparable to the related distances (1.93 to 2.14 Å) found in the literature. *See, e.g.,* Hollis et al., J. Am. Chem. Soc. 105:4293-4299 (1983), which discloses $[Au(terpy)Cl]^{2+}$ (terpy = 2,2',2"-terpyridine); Liu et al., J. Chem. Soc., Chem. Comm. 17 :1787-1788 (1995), which discloses $[Au(C\text{^}N\text{^}N)Cl]^+$ (C^N^N = 6'-phenyl-2,2'-bipyridine); and Calamai et al., Inorg. Chim. Acta 285:309-312 (1999), which discloses $[Au(esal)]^{2+}$ (esal = *N*-ethylsalicylaldiminate).

Table 1. X-ray Crystallographic Data for Complex **1a**.

| | |
|---|---|
| empirical formula | $C_{44}H_{28}AuClN_4O_4$ |
| formula weight | 909.12 |
| temperature | 253(2) K |
| wavelength | 0.71073 Å |
| crystal system | Orthorhombic |

(continued)

| | | |
|---|---|---|
| space group | P n n a | |
| unit cell dimensions | $a$= 8.1020(16) Å | $\alpha$= 90° |
| | $b$= 20.964(4) Å | $\beta$= 90° |
| | $c$= 20.060(4) Å | $\gamma$= 90° |
| volume | 3407.2(12) Å | |
| Z | 4 | |
| density (calculated) | 1.772 $mg/m^3$ | |
| absorption coefficient | 4.451 $mm^{-1}$ | |
| $F$(000) | 1792 | |
| crystal size | 0.4 x 0.1 x 0.1 $mm^3$ | |
| theta range for data collection | 1.40 to 25.63° | |
| index ranges | -9<=h<=9, -24<=k<=25, -24<=1<=24 | |
| reflections collected | 13426 | |
| independent reflections | 3006 [R(int) = 0.0478] | |
| completeness to theta = 25.63° | 95.3 % | |
| absorption correction | None | |
| refinement method | Full-matrix least-squares on $F^2$ | |
| data / restraints / parameters | 3066 / 0 / 248 | |
| goodness-of-fit on $F^2$ | 0.934 | |
| final $R$ indices [I>2sigma(I)] | $R_1$ = 0.0308, w$R_2$ = 0.0790 | |
| $R$ indices (all data) | $R_1$ = 0.0645, w$R_2$ = 0.1040 | |
| largest diff. peak and hole | 0.716 and -1.590 $e$ $Å^{-3}$ | |

Table 2. Selected Bond Distances (Å) and Bond Angles (deg) for Complex **1a.**

| | Bond Distances (Å) |
|---|---|
| Au-N(1) | 2.032(5) |
| Au-N(1*) | 2.032(5) |
| Au-N(2*) | 2.033(5) |
| Au-N(2) | 2.033(5) |
| | Bond Angles (deg) |
| N(1)-Au-N(1*) | 89.8(3) |
| N(1)-Au-N(2*) | 177.31(19) |
| N(1*)-Au-N(2*) | 90.1(2) |
| N(1)-Au-N(2) | 90.1(2) |
| N(1*)-Au-N(2) | 177.31(19) |
| N(2*)-Au-N(2) | 90.1(3) |

*Gold(III) Complexes 2 (Schiff base) and 3c (Bis(pyridyl)carboxamide):* Gold(III) Complexes **2a** and **3c** were prepared by reacting $K[Au^{III}Cl_4]$ with the corresponding ligands in refluxing acetonitrile (fo**r 2a**) and acetic acid (for **3c**) according to the reported procedures *(see* Barnholtz et al, Inorg. Chem. 40: 972 - 976, (2001)). The complexes were characterized by $^1$H-NMR and FAB-MS spectroscopies.

Complex **2a:** FAB-MS: $mlz$ = 463 ($M^+$).

Complex **3c:** FAB-MS: $mlz$ = 694 ($M^+$)].

**Example 2. Stablity Studies of the Gold(III) Complexes**

Example 2 describes the results of stability studies for illustrative Gold(III) Complexes **1a**, **1b**, **1c**, **1d**, **1e**, **2a** and **3c.**

*UV-vis experiments.* For UV-vis analysis, the absorption spectra of 2.5 $\mu$M (7 nmoles) of all studied complexes in (1: 19) MeCN/ Tris buffer solution was monitored over 72 h. A solution of Tris buffer/ MeCN (19:1) solution and Complex

**1a** reveals an intense Soret absorption band at $\lambda_{max}$ = 410 nm ($\varepsilon$= 240,000 $dm^3mol^{-1}cm^{-1}$) and a weaker Q band at $\lambda_{max}$ = 520 nm. The gold(III) solution was monitored over 48 h at room temperature and less than 10% decrease in the Soret band intensity was observed without any significant spectral shift in both the Soret and Q-bands. The results are shown in FIG. 3.

However, in the presence of glutathione (2 mM in Tris buffer/ MeCN = 19:1), the Soret band intensity of Complex **1a** was found to decrease by about 27% with concomitant band broadening upon standing at room temperature over 48 h (FIG. 4). Yet, no significant spectral shift and formation of new absorption bands were observed. Careful examination of the Q band region did not reveal any absorption peaks corresponding to the presence of the free base porphyrin ($\lambda_{max}$ = 514, 548, 590 and 640 nm), and thus demetallation can be excluded. Unlike Complex **1a**, the UV-vis spectrum of Complex **1j** did not show any significant changes over 72 h under an identical buffer medium containing glutathione (2 mM), *i.e.,* no significant changes in the Soret band intensity and band shape were observed.

UV-vis studies also showed that Complexes **1j** and **1k** were stable in Tris buffer saline (TBS; pH 7.2) for over 48 h at room temperature. Moreover, no significant UV-vis spectral changes were observed when treating **1j/1k** with excess GSH. This finding confirms the excellent solution stability of **1j** and **1k** in physiologically related medium.

Likewise, TBS-MeCN (9:1) (pH 7.2) solutions of the Schiff-base (**2a**) and bis(pyridyl)carboxamide (**3c**) complexes exhibited no significant spectral changes after standing at room temperature for 4 h. The results indicates that the gold(III) Schiff-base (**2a**) and bis(pyridyl)carboxamide complexes are stable. However, addition of GSH to the TBS solutions of Complexes **2a** and **3c** resulted in spontaneous spectral changes, accompanied by formation of some colloidal gold and light yellow precipitates. The precipitates are soluble in common organic solvent such as THF, $CHCl_3$ and DMSO but insoluble in aqueous solution. FAB-MS analysis of the precipitates revealed the molecular ions of the free $H_2$Salen (*m*/*z* = 267) for Complex **2a** and $H_2N_4$ *(m*/*z* = 498) for Complex **3c.** And yet, ESI-MS analysis of the remaining solutions did not reveal any ion species corresponding to the molecular ions of Complexes **2a** *(mlz* = 463) and **3c** *(mlz* = 694). The results indicate that Complexes **2a** and **3c** underwent extensive demetallation and reduction of gold(III) to colloidal gold upon reaction with GSH.

*$^1$H-NMR experiments.* NMR experiments were carried out with a $d_3$-MeCN/$D_2O$ (1: 9) solution of Complex **1a** (0.5 mM) in the presence of 4 mole equivalent of GSH as described by Sun et al., Eur. J. Biochem. 267:5450-5457 (2000). The pH was adjusted to 7.4 with NaOD, and the solution was degassed for 10 min by bubbling with nitrogen to minimize oxidation of GSH. Similar experimental setup was done for Complex **1j**, except this analysis was carried out in $D_2O$ only. The electronic spectra of the resulting mixtures was then monitored for 72 h.

The mixture of Complex **1a**/ GSH (2 mM) mixture was monitored by $^1$H NMR in $D_2O$/ $CD_3CN$ mixture (9:1 v/v), and no significant spectral changes were observed for over 72 h at room temperature. It is known that the chemical shifts of the pyrrolic protons are sensitive to the oxidation states of the gold center, and reduction of Au(III) to Au(I) or Au(0) is expected to be accompanied by spectral changes in the pyrrolic region. However, in this work, we did not observe any significant changes of the spectral pattern in the aromatic region ($\delta_H$ 7.2 - 8.4 ppm) for Complexes **1a** and **1j,** and therefore reduction of Au(III) to Au(I) or Au(0) by GSH is untenable. This finding strongly suggests that the gold (III) porphyrin is stable in physiological conditions, even in the presence of mild reducing agents.

Similarly, no significant $^1$H NMR spectral changes were observed when treating **1j/1k** with excess GSH. This finding confirms the excellent solution stability of **1j** and **1k** in physiologically related medium.

Glutathione can be oxidized to form a disulfide bridged dimer (GSSG) with a mild oxidizing agent such as gold(III) complex. A previous study by Sun et al., Eur. J. Biochem. 267:5450-5457 (2000) established that GSSG is characterized by a doublet absorption peak at $\delta_H$ 3.30 ppm, assignable to $\beta$-$CH_2$ group as depicted in Scheme 5.

Scheme 5. Characteristic proton resonance absorptions for (a) GSH and (b) GSSG in $D_2O$

a)

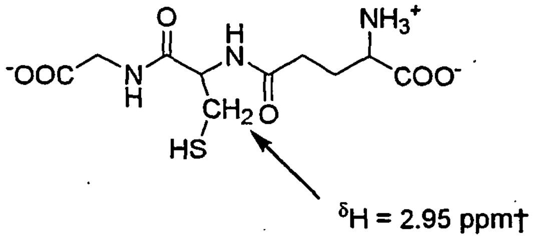


b)

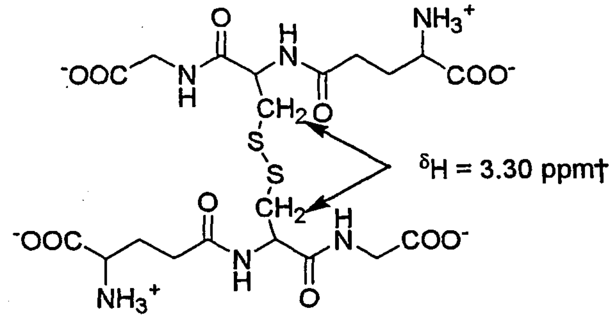


† Data obtained from: Sun et al., *J. Biochem.* 267:5450 (2000).

In our $^1H$ NMR study, we did not observe any doublet proton absorption around the 3.30 ppm region corresponding to the GSSG formation or binding of GSH to the gold(III) center. The results of the NMR studies indicate that illustrative Complexes 1a-1k are stable under physiologically relevant conditions. The result indicates that the Gold(III) Complexes of the invention are stable under physiologically relevant conditions

With regard to the Soret band broadening as noted in Section above, we found that addition of acetone (0.4 ml) to the Tris buffer solution containing Complex **1a** led to complete recovery of the Soret band to its initial band shape without any changes in the extinction coefficient (512,000 $dm^3mol^{-1}cm^{-1}$). We reason that the observed band broadening is probably due to aggregation of the gold(III) porphyrin complex in aqueous buffer solution.

*Electrochemical measurements.* Electrochemical measurements were performed at room temperature after purging with nitrogen using 0.1 M tetrabutylammonium hexafluorophosphate (TBAP)/acetonitrile as supporting electrolyte. The working electrode was a glassy carbon (Atomergic Chemetal V25, geometric area of 0.35 $cm^2$) electrode and the counter electrode was platinum gauze. A nonaqueous $Ag/AgNO_3$ (0.1 M in acetonitrile) reference electrode was contained in a separate compartment connected to the test solution via fine sintered glass disks. The ferrocenium/ ferrocene couple was used as the internal standard. The redox properties of the gold(III) porphyrin complexes in nonaqueous medium ($CH_2Cl_2$ with 0.1 M $nBu_4NPF_6$) have been studied by cyclic voltammetry with glassy carbon and platinum as the working and counter electrodes, respectively. FIG. 5 shows the cyclic voltammogram of Complex **1a,** which exhibits two reversible reduction waves at -1.00 and -1.48 V vs $Cp_2Fe^{+/0}$. Table 3 shows the electrochemical data for Complexes **1e**, **1d**, **1a**, **1b** and **1c**, *i.e.*, tetrakis(*para*-Y-substituted phenyl)porphyrins where Y = -Cl , -Br, -H, -Me and -MeO, respectively. Apparently, electron-withdrawing groups such as -Br- and -Cl promote reduction of the gold(III) porphyrin as reflected by a smaller $E^0$ values for Complexes **1d** and **1e,** respectively. Complexes **1b** and **1c** with electron-donating groups, *i.e.*, -methyl and - methoxy, respectively, display larger $E^0$ values, indicating that reduction is less favored. Interestingly, both the reduction couples I and II show a parallel substituent effect on their electrochemical potential values. This can be illustrated by plotting the $\Delta 4E°$ ($\Delta E° = E°_Y - E°_H$) values of the couples against each other over a series of *para*-substituents; a straight line with a slope of 1.00 was obtained (graph not shown). The effect of para-substitution on the electrochemical potentials has been examined by Hammett correlation analysis. Fitting (by least-squares method) the $\Delta E°$ values with the $4\sigma p$ values, where $\sigma_p$ is the Hammett substituent constant, a straight line ($R$ = 0.97) was obtained, albeit with some deviation from linearity.

Table 3. Reduction Potentials Complexes of Formula (I), containing -phenyl substituents.

| entry | Complex | Couple I[a]/V | Couple II [a]/V | $\Delta E_1^o$ [b]/V | $\Delta E_2^o$ [b]/V | $\sigma_{JJ}$ [c] | $\sigma_p$ [d] |
|---|---|---|---|---|---|---|---|
| 1 | **1e** | -0.96 | -1.44 | +0.44 | +0.44 | 0.22 | 0.34 |
| 2 | **1d** | -0.97 | -1.45 | +0.03 | +0.03 | 0.23 | 0.26 |
| 3 | **1a** | -1.00 | -1.48 | 0 | 0 | 0 | 0 |
| 4 | **1b** | -1.01 | -1.49 | -0.01 | -0.01 | 0.15 | -0.17 |
| 5 | **1c** | -1.02 | -1.51 | -0.02 | -0.02 | 0.23 | -0.27 |

[a] Couple I and couple II are the first and the second reduction couples of $[Au^{III}(p\text{-}Y\text{-}TPP)]Cl$ Complexes, respectively. [b] $\Delta E° = E^o_Y - E^o_H = \rho_{JJ}{}^{\cdot}\Sigma\sigma_{JJ}{}^{\cdot} + \rho_p\Sigma\sigma p = 0.00649\ \Sigma\sigma_{JJ}{}^{\cdot} + 0.0238\ \Sigma\sigma_p$ ($|\rho_p/\ \rho_{JJ}{}^{\cdot}| = 3.67$). [c] See ref. Jiang, X.-K. Acc. Chem. Res. 1997, 30, 283-289. [d] See *ref.* Isaacs, N. S. Physical Organic Chemistry, Longman Scientific & Technical. Harlow, Essex, England 1995.

The results indicate that the electronic characteristics of the illustrative Gold(III) Complexes can be "tuned" by appending different groups to the ligand, implying that the electronic characteristics of the Gold(III) Complexes can be tuned to improve their effectiveness as inhibitors of reverse transcriptase of Human Immunodeficiency virus-1 or as anti-tumor agents.

## Example 3. Cytotoxicity Studies using the Gold(III) Complexes

Example 3 describes the results of cytotoxicity studies using illustrative Gold(III) Complexes **1a-1k, 2a-2c** and **3a-3c**.

The anti-cancer activities of the gold(III) porphyrin complexes were evaluated toward some established human cancer cell lines: promyelocytic leukemia (HL-60), hepatocellular carcinoma (HepG2), cervix epitheloid carcinoma (HeLa), epidermoid carcinoma (KB-3-1) and its multi-drug resistant variant (KB-V1), nasopharyngeal carcinoma (SUNE1) and its cisplatin-resistant variant (CNE1). The experiments were carried out by following the MTT procedure (*see* Mosmann et al., J. Immunol. Methods 65:55-63 (1983). The $IC_{50}$ values of the gold(III) porphyrin complexes together with that of cisplatin, and $KAuCl_4$ are provided in Table 4a; and the $IC_{50}$ values of the gold(III) Schiff-base and bis(pyridyl) carboxamide complexes are shown in Table 4b. The cytotoxicity profiles of some selected gold(III) porphyrin complexes toward SUNE1 and its cisplatin-resistant variant CNE1 are also shown in FIG. 7.

Table 4a. *In Vitro* Growth Inhibition of Selected Cancer Cell Lines by Gold(III) Porphyrin Complexes (a) and Gold(III) Schiff-base and Bis(pyridyl)carboxamide Complexes (b).

| (a) | | $IC_{50}$ (μM | | | | | | |
|---|---|---|---|---|---|---|---|---|
| **entry** | **Complex** | **KB-3-1 [a]** | **HL-60** | **HepG2** | **SUNE1** | **HeLa** | **KB-V-1** | **CNE1** |
| 1 | **1a** | 0.20 | 0.73 | 0.34 | 0.11 | 0.28 | 0.12 | 0.17 |
| 2 | **1b** | 0.41 | 1.53 | 1.11 | 1.09 | 0.52 | 1.15 | 0.99 |
| 3 | **1c** | n.d.[b] | 0.88 | 0.76 | 0.68 | 0.66 | n.d.[b] | 0.77 |
| 4 | **1d** | 1.29 | 1.12 | 1.21 | 0.74 | 0.89 | 0.64 | 0.98 |
| 5 | **1e** | 0.50 | 0.87 | 0.92 | 0.34 | 0.69 | 0.23 | 0.41 |
| 6 | **1f** | 0.87 | 0.74 | 1.83 | 0.68 | 0.68 | 0.71 | 0.73 |
| 7 | **1g** | n.d.[b] | 1.45 | 2.31 | 2.11 | 3.18 | n.d.[b] | 1.45 |
| 8 | **1h** | 0.99 | 0.75 | 1.17 | 1.18 | 0.96 | 1.14 | 2.06 |
| 9 | **1i** | 0.91 | 1.24 | 1.09 | 0.96 | 1.06 | 0.99 | 1.37 |
| 10 | **1j** | > 50 | > 50 | > 50 | > 50 | > 50 | > 50 | > 50 |
| 11 | **1k** | > 50 | > 50 | > 50 | > 50 | > 50 | > 50 | > 50 |
| 12 | $n\text{Bu}_4\text{N}[\text{AuCl}_4]$ | 10.3 | 9.71 | 11.2 | 9.94 | 8.41 | n.d.[b] | 11.5 |
| 13 | $Pt(NH_3)_2Cl_2$ | 13.2 | 16.8 | 13.5 | 12.6 | 11.2 | 10.2 | 40.8 |
| 14 | [Zn(TPP)] | > 50 | > 50 | > 50 | > 50 | > 50 | n.d.[b] | > 50 |

| (b) | | $IC_{50}$ (μM) |
|---|---|---|
| entry | Complex | HeLa |
| 1 | **2a** | 11.8 |
| 2 | **2b** | 9.71 |
| 3 | **2c** | 16.5 |
| 4 | **3a** | 33.9 |
| 5 | **3b** | 26.2 |
| 6 | **3c** | 10.8 |
| 7 | $Pt(NH_3)_2Cl_2$ | 11.2 |

[a]Human Oral Epidermoid Carcinoma (KB-3-1); Human Promyelocytic Leukemia (HL-60); Human Hepatocellular Carcinoma (HepG2); Human Nasopharyngeal Carcinoma (SUNE1); Human Cervix Epitheloid Carcinoma (HeLa); Human Oral Epidermoid Carcinoma (multi-drug resistance, KB-V-1); Human Nasopharyngeal Carcinoma (cisplatin resistance, CNE1).
[b]n..d. = not determined

All the gold(III) porphyrin complexes studied in this work, except Complexes **1j** and **1k**, show relevant cytotoxic activities with $IC_{50}$ values falling into the micromolar range (0.1-15 μM). Among them, Complex **1a** was found to be the most cytotoxic with the smallest $IC_{50}$ values of 0.1-0.8 μM.

The cytotoxic properties of the [$Au^{III}$(*para*-Y-TPP)]Cl Complexes **1e**, **1b**, **1a**, **1d** and **1e** (Y = MeO, Me, H, Br and Cl, respectively) were examined, and their cytotoxicities are largely unaffected by electronic substituent effect.

Compared to the other gold(III) porphyrins, Complexes **1j** and **1k** are practically non-cytotoxic as manifested by their rather large $IC_{50}$ values > 50 μM. This may be ascribed to the highly polar and hydrophilic groups that rendered the compounds unable to pass through the hydrophobic cell membrane.

In this study, all the gold(III) porphyrin complexes excepted higher cytoxicities toward the studied cell lines than cisplatin. This is shown in Table 5, where the ratio of Cisplatin $IC_{50}$/ Gold(III) Porphyrin Complex $IC_{50}$ for a given cell line.

Table 5. $IC_{50}$ Ratio [Cisplatin / Gold(III) Porphyrin Complexes]

| | | $IC_{50}$ ratio [cisplatin / gold(III) porphyrin complexes] | | | | |
|---|---|---|---|---|---|---|
| entry | Compound | KB-3-1 | HeLa | SUNE1 | KB-V1 | CNE1 |
| 1 | **1a** | 17.8 | 40.0 | 114.5 | 25.1 | 240.2 |
| 2 | **1b** | 32.1 | 21.5 | 11.5 | 18.4 | 41.2 |
| 3 | **1c** | nd.[a] | 17.0 | 18.5 | n.d.[a] | 53.0 |
| 4 | **1d** | 10.23 | 12.6 | 17.0 | 8.96 | 41.6 |
| 5 | **1e** | 26.4 | 16.2 | 37.1 | 20.4 | 99.5 |
| 6 | **1f** | 15.1 | 16.5 | 18.5 | 5.62 | 55.9 |
| 7 | **1g** | nd.[a] | 3.52 | 5.97 | nd.[a] | 28.1 |
| 8 | **1h** | 13.3 | 11.7 | 10.7 | 6.12 | 19.8 |
| 9 | **1i** | 14.5 | 10.56 | 13.1 | 7.31 | 29.8 |

[a] n.d.=not determined

The results in Table 5 also show that except for Complexes **1i, 1j** and **1k,** the gold(III) porphyrin complexes show at least ten times higher cytotoxicity than cisplatin. Notably, Complex **1a** exhibits significant cytotoxicity ($IC_{50}$ = 0.17 μM) against the cisplatin-resistant nasopharyngeal carcinoma (CNE1) cell lines; the ratio of the $IC_{50}$ values for Complex **1a** and cisplatin was determined to be 240 (see Table 5). The important role of the gold(III) ion on the cytotoxic

properties of the metalloporphyrin is reflected by the fact that the [$Zn^{II}TPP$] complex is at least 100-fold less cytotoxic ($IC_{50}$ > 50 μM) than Complex **1a** against an identical panel of cell lines. By comparing the cytotoxicity profile of Complex **1a** with that of $KAu^{III}Cl_4$, the porphyrin ligand appears to have a significant role in promoting the cytotoxicity of gold(III) complex. The results indicate that $KAu^{III}Cl_4$ is about 40 times less potent than Complex 1a in killing oral epidermoid and nasopharyngeal cancer cells. As noted earlier, the Au(3+) ion is unstable under physiological conditions and undergoes reduction to colloidal gold. Without being bound by theory, we believe that the porphyrin ligand stabilizes the Au(3+) center and serves as a carrier to bring the toxic metal to its cellular target.

The cytotoxicities of the other gold(III)porphyrin complexes toward the cisplatin-resistant cell line (CNE1) and the cisplatin-sensitive cell lines (SUNE1) the cisplatin-resistant cell line (CNE1) and the cisplatin-sensitive cell lines (SUNE1) are similar to that of Complex **1ar**; the ratio of $IC_{50}$ (CNE1/ SUNE1) values are close to unity. The results suggests that the mechanism for the cytotoxicity of the gold(III) porphyrin complexes is different from that of cisplatin.

**<u>Example 4. Interaction of Gold(III) Porphyrin Complexes with DNA.</u>**

Example 4 describes the results of studies showing the interaction of illustrative Gold(III) Complexes **1a**, **1g**, **1h** and **1j** with DNA.

*Restriction Endonuclease Fragmentation Assay:* Restriction endonuclease fragmentation assay was used to detect potential interaction of Complex **1a** with DNA. Binding of the metalloporphyrin onto a duplex DNA such as plasmid DNA (pDR2) is expected to occupy the binding site of the restriction enzyme ApaI, thereby inhibiting the enzymatic DNA digestion. *See* Ikeda et al., J. Am. Chem. Soc. 104:296-297 (1982).

FIG. 8 shows the results of electrophoresis of pDR2 after restriction enzyme digestion in the absence and presence of Complex **1a.** The undigested DNA (lane A) shows a fragment at around 9.6 kbp corresponding to the circular DNA. After ApaI digestion, (lane B), three bands corresponding to 6.6, 4.4 and 2.0 kbp were observed. In the presence of Complex **1a** at 1:1 (Complex **1a** :DNA bp) ratio, inhibition of the DNA digestion was observed (lane D). Yet, partial DNA digestion was observed at a lower molar concentration of Complex **1a** (lane C) (Complex **1a**:DNA = 0.1:1). For comparison, the presence of ethidium bromide (a classical intercalator; lane F) and Hoechst 33342 (a minor groove binder; lane H) led to complete inhibition of the DNA digestion. The results suggest that Complex **1a** would bind to DNA and inhibit enzymatic digestion by restriction endonucleases.

*Absorption Titration.* The binding interaction of the gold(III) porphyrins to duplex DNA has been studied by UV-vis spectroscopy. As shown in FIG. 9, addition of calf thymus DNA (0-5 μM) to Complex **1a** in a Tris buffer solution containing 2% DMSO induced a significant hypochromicity (44%) of the Soret band, accompanied with a small bathochromic shift of 5 nm. For the Q-band at 520 nm, 40% hypochromicity and 6 nm bathochromic shift were also observed. It is noteworthy that the spectral changes displayed clear isosbestic points at 290, and 425 nm, which remain unchanged throughout the spectroscopic titration experiment.

Inset A of FIG. 9 shows the plot of $I_o/I$ *versus* [ctDNA]/[**1a**], where A and A are the Soret band absorbances of Complex **1a** in the absence and presence of ctDNA, respectively. As shown in Inset A, the $A_o/A$ value increases linearly with the rising [ctDNA]/[ Complex **1a**] ratio from 0 to 1.0; no further hypochromicity of the Soret band was observed when the [ctDNA]/[ Complex **1a**] is larger than 1.0. This result indicates that the binding stoichiometry (Complex **1a**:number of base pairs) is 1:1.

The intrinsic binding constant ($K_b$) for Complex **1a** toward calf thymus DNA was determined according to equation 1:

$$\frac{[\text{ctDNA}]}{\Delta\varepsilon_{ap}} = \frac{[\text{ctDNA}]}{\Delta\varepsilon} + \frac{1}{(\Delta\varepsilon \times K_b)} \quad (1)$$

where [ctDNA] is the concentration of calf thymus DNA, $\Delta\varepsilon_{op}=|\varepsilon_A-\varepsilon_B|$ in which $\varepsilon_A=A_{obs}$ [complex], and $\Delta\varepsilon=|\varepsilon_B-\varepsilon_F|$ and correspond to the extinction coefficient of DNA-bound gold(III) porphyrin and the free gold(III) porphyrins, respectively. Using the absorbance data of the Soret band, the plot of [ctDNA]/$\Delta\varepsilon_{ap}$ vs [ctDNA] gives a straight line (R = 0.99) with a slope of 9.15 x $10^{-6}$, which corresponds to the reciprocal of the $\Delta\varepsilon$ value. From the intercept of the plot, the intrinsic binding constant for Complex **1a** to calf thymus DNA was determined to be (2.79 ± 0.34) x $10^6$ $dm^3mol^{-1}$.

Similarly, Complexes **1g, 1h** and **1j** also produced comparable isosbestic spectral changes upon interaction with calf thymus DNA (data not shown). Significant hypochromicity (20 - 50 %) and bathochromic shift (3-6 nm) of the Soret bands were observed. On the basis of the $A_o/A$ vs [ctDNA]/ [metal complex] plot, the stoichiometric binding ratio for the gold(III) complexes with the number of base pairs were also found to be 1:1. Again using the equation 1, their intrinsic binding constants to calf thymus DNA were determined, and the values are tabulated in Table 6.

Table 6. DNA Binding Parameters for Selected Classes of Gold(III) Porphyrin Complexes in Tris Buffer at Various Temperatures

| Complex | temperature, *K* | intrinsic binding constant ($K_b$), $dm^3mol^{-1}$ | hypochromicity, % |
|---|---|---|---|
| **1a** | 292.8 | $2.79 \times 10^6$ | 53.4 |
| | 298.0 | $2.14 \times 10^6$ | 50.3 |
| | 302.3 | $1.98 \times 10^6$ | 49.5 |
| | 307.2 | $1.55 \times 10^6$ | 47.1 |
| | 311.6 | $1.35 \times 10^6$ | 46.3 |
| | 316.5 | $0.94 \times 10^6$ | 43.4 |
| | 321.9 | $0.82 \times 10^6$ | 42.5 |
| **1g** | 291.8 | $0.73 \times 10^6$ | 18.2 |
| | 297.6 | $0.58 \times 10^6$ | 18.0 |
| | 301.4 | $0.47 \times 10^6$ | 16.5 |
| | 306.1 | $0.42 \times 10^6$ | 10.1 |
| | 311.7 | $0.34 \times 10^6$ | 8.7 |
| **1h** | 291.4 | $4.14 \times 10^6$ | 30.1 |
| | 296.8 | $2.73 \times 10^6$ | 29.2 |
| | 302.2 | $1.51 \times 10^6$ | 28.0 |
| | 305.5 | $1.27 \times 10^6$ | 26.1 |
| | 311.2 | $0.96 \times 10^6$ | 24.4 |
| **1J** | 291.3 | $0.49 \times 10^6$ | 47.8 |
| | 297.2 | $0.36 \times 10^6$ | 45.9 |
| | 303.7 | $0.24 \times 10^6$ | 43.1 |
| | 307.2 | $0.21 \times 10^6$ | 41.1 |
| | 311.9 | $0.19 \times 10^6$ | 40.6 |

The intrinsic binding constants obtained in this work are comparable to reported values ($10^4$ to $10^6$ $dm^3mol^{-1}$) of some related metalloporphyrin complexes disclosed by Lugo-Ponce et al., Coord. Chem. Rev. 208:169-191 (2000); Tjahjono et al.; J. Inorg. Biochem. 85:219-228 (2001); and Han et al., J. Inorg. Biochem. 91:230-236 (2002).

In a control experiment using sodium dodecyl sulfate (0 - 5 μM) instead of DNA, similar UV-vis spectral changes for Complexes **1a** and **1g** were observed (48 % hypochromicity and 6 nm bathochromic shift) (data not shown). This result suggests that the observed spectral changes with the calf thymus DNA is probably due to electrostatic binding of the cationic metal complexes with the polyanionic DNA phosphate backbone. *See* Neidle, S. DNA structure and recognition; Oxford University Press: Oxford, 1994; 74-79. However, when Complexes **1h** and **1j** was titrated with sodium dodecyl sulfate, minor hypochromicity (< 10%) of their Soret bands was observed, suggesting that other binding modes for the gold complexes may be more important than electrostatic binding with the DNA backbone.

The effect of temperature on the intrinsic binding constants was investigated. Using the van't Hoff equation (2):

$$\ln K = -(\Delta H/RT) + (\Delta S/R) \qquad (2)$$

the linear plot of 1n K *vs* 1/T yields the thermodynamic parameters for the DNA binding interactions. As shown in Table 7, the DNA binding interactions with Complexes **1a**, **1g**, **1h** and **1j** show a comparable negative ΔH value (-7 to -13 $kcalmol^{-1}$), indicative of favorable enthalpic changes.

Table 7. Thermodynamic Parameters for the Binding of Complexes **1a**, **1g**, **1h** and **1j** to Calf Thymus DNA

| entry | Complex | ΔG° (298 K) | ΔH° | ΔS° |
|---|---|---|---|---|
| 1 | **1a** | -8.7±0.4 | -8.0±0.5 | +2.4±0.3 |
| 2 | **1g** | -7.8±0.5 | -7.0±0.4 | +2.7±0.7 |
| 3 | **1h** | -8.2±0.7 | -13.8±1.2 | -17.3±0.7 |
| 4 | **1j** | -7.5± 0.4 | -8.5±0.6 | -3.4±0.5 |

However, Table 7 shows that there are notable variations for the ΔS values among the gold(III) complexes. For Complexes **1a** and **1g,** the DNA binding reactions are associated with small positive entropic changes of +2.4 and +2.7 calmol$^{-1}$K$^{-1}$, respectively, consistent with increasing disorder upon binding of the complexes to DNA. On the other hand, the binding of DNA to Complexes **1h** and **1j** produces negative entropic changes (-17.3 cal mol$^{-1}$ K$^{-1}$ (Complex **1h**), **-**3.4 calmol$^{-1}$K$^{-1}$ (Complex **1j**)), indicating a loss of degree of freedom upon binding to DNA. This finding suggests that different binding modes would be expected for these two groups of gold(III) complexes.

*Viscosity evaluation.* The binding interaction of the gold(III) porphyrins can be further investigated by examining the change of DNA viscosity upon binding of the metalloporphyrin. If the metalloporphyrin binds to DNA by intercalation, this would cause chain elongation, unwinding and stiffening of the DNA helix as a consequence of untwisting of the base pairs and helical backbone needed to accommodate the intercalator.

Based on the theory of Cohen and Eisenberg (Cohen et al, Biopolymers 8:45-55 (1969)), the viscosity data were plotted as $(\eta/\eta_o)^{1/3}$ the binding ratio, r = [DNA]/ [Complex **1a**]. The plot is shown in FIG. 10. For comparison, the viscosity profiles for ethidium bromide (intercalator) and Hoechst 33342 (minor groove binder) are also shown is FIG. 10. As anticipated, the intercalator, *i.e.*, ethidium bromide, increases the viscosity of the DNA by increasing its hydrodynamic length. Yet, both Complex **1a** and Hoechst 33342 do not lengthen the DNA and exhibit no appreciable change in viscosity of the DNA. The result suggests that Complex **1a** is unlikely to interact with DNA through intercalation.

*Gel mobiliy shift assay.* Additional evidence for non-intercalative binding of Complex **1a** to DNA was obtained by gel mobility shift assay. Elongated DNA will have lower mobility compared to the free DNA in the agarose gel electrophoresis. FIG. 11 shows the results of the gel mobility shift assay. The 100-bp PCR, alone or treated with Complex **1a**, Hoechst 33342 (H33342) and ethidium bromide, were mixed and resolved by agarose gel electrophoresis. It is apparent that those DNA samples treated with H33342 (lane C) and Complex **1a** (lane D and E) showed similar mobility shift to the untreated 100-bp PCR. In contrast, lane B showed the tailing effect, which was due to the intercalation of the ethidium bromide to the DNA base pairs. The result suggests that Complex **1a** is unlikely to bind to DNA by intercalation.

**Example 5. Apoptosis Studies.**

Example 5 describes the results of a cell morphology study using illustrative Gold(III) Complex **1a.**

*Confocal Microscopic Experiment.* By examining cell morphology using scanning confocal microscopy, the type of cell death can be recognized. The early phase of apoptosis is characterized by the formation of apoptotic bodies while the integrity of the cell membrane is maintained without cell lysis. On the contrary, necrosis is characterized by cell lysis with the internal cellular components such as DNA being exposed. Using suitable staining agents (ethidium bromide (EB) for DNA and acridine orange (AO) for cell membrane) normal, necrotic and apoptotic cells can be distinguished by laser confocal microscopy. *See* Chan et al., Inorg. Chem. 41:3161-3171 (2002).

The picture of the cells is depicted in FIG. 12. After incubation of the HeLa cells with Complex **1a** for 15 h, living cells and apoptotic cells were observed, and several apoptotic bodies can be seen (marked by circles) in several cells. Importantly, no cellular DNA was exposed due to necrosis.

*Flow cytometry:* The gold(III) porphyrin-induced apoptosis of the HeLa cell line has been further examined by flow cytometry. During the early phase of apoptosis, cells lose the asymmetry of their membrane phospholipids, although the integrity of the cell membrane is maintained. A negatively charged phospholipid, named phosphatidylserine (PS), which is located in the inner part of the plasma membrane, becomes exposed to the cell surface. A phospholipid-binding protein, annexin V (green emissive upon binding), with high affinity binds preferentially to PS. Apoptotic cells therefore can be stained by annexin V before the dying cell changes its morphology and hydrolyzes its DNA. On the other hand, both DNA and the PS would be exposed for necrotic cells. In this case, propidium iodide (PI, red emissive upon binding), can be used to stain selectively the naked DNA. Therefore, both apoptotic and necrotic cells can be discriminated after adding these two staining reagents. For apoptotic cells, only annexin V would stain; for necrotic cells, both annexin V and PI would stain.

In this experiment, annexin V and PI were added to cell cultures that had either already exposed to Complex **1a** (6 and 15 h) or staurosporine (15 h). For comparison, a control cell culture experiment was performed in the absence of Complex **1a** and staurosporine. The results (Figure 13a) show that in the absence of any drug compounds, more

than 90% of the cells were neither stained by annexin V (x-axis emission) nor PI (y-axis emission), indicating that over 90% of the population were living cells. Upon treatment with Complex **1a** or staurosporine, the magnitude of the annexin V emission was enhanced, indicating the onset of apoptosis. The progress of apoptosis induced by Complex **1a** was observed by monitoring the magnitude of the annexin V emission. The results in FIG. 13 show that 13, 24 % of the cells underwent apoptosis after 6h (13c,g) of incubation; 57 % of the cells died via apoptosis after 15h (13d,h) of incubation. Throughout the flow cytometric experiment, <1% of necrotic cells were observed.

Irrespective of the mechanism for induction of apoptosis, the results of the confocal microscopy and flow cytometry study show that Complex **1a**, an illustrative Gold(III) Complex of the invention, can induce apoptosis in HeLa cell. Without being limited by theory, it is believed that the treatment of the cancer cells with Complex **1a** "nicks" cellular DNA and causes oxidative stress. The results indicated that the Gold(III) Complexes are useful for induction of apoptosis in cancer cells.

**Example 6. Inhibition of HIV-1 Reverse Transcriptase by Gold(III) Porphyrins and Derivatives.**

Example 6 describes a study showing that illustrative Gold(III) Complexes **1a**, **1j**, **1k**, **2a** and **3c** are useful for inhibiting HIV-1 RT activity.

HIV-1 reverse transcriptase activities were measured by an ELISA method (*see* Eberle et al, J. Virol. Methods, 40:347-356 (1992) performed by incubating the enzyme with digoxigenin-labeled deoxyuridin-5'-triphosphate (dUTP) and the biotin-labeled analogue during reverse transcription starting from the template/ primer hybrid poly(A) •oligo$(dT)_{15}$. After reverse transcription, the newly synthesized DNA was immobilized onto streptavidin-coated ELISA wells, and the incorporation of the dioxigenin-labeled dUTP was assayed by chemiluminescence method. For example, treatment of HIV-1 RT in Lysis buffer (2 ng, 128.7 μL) with Complex **1j** (1.3 μl of 5 mM stock solution) dissolved in TBS at 37°C produced significant RT activity inhibition (~95%) after 30 min incubation. For comparison, the drug-free control exhibited 0% inhibition. The results of the study are shown in Table 8.

Table 8. Inhibitory Concentration ($IC_{50}$) of Illustrative Gold(III) Complexes Against HIV-1 RT and Human Lung Fibroblast Cell Line

| entry | Complex | $IC_{50}$/μM (± SE)[a] HIV-1 RT | $IC_{50}$/μM (± SE)[a] CCD-19Lu |
|---|---|---|---|
| 1 | **1a** | 28.4 ± 1.8 | 0.91±0.21 |
| 2 | **1j** | 0.31 ± 0.05 | > 100 |
| 3 | **1k** | 0.57 ± 0.09 | > 100 |
| 4 | **2a** | 0.33 ± 0.03 | 38.6 ± 4.43 |
| 5 | **3c** | 0.47 ± 0.09 | 43.9 ± 3.25 |
| 6 | AZT | 0.069 ± 0.008 | n.d. |

[a] Expressed as mean ± SE of at least three determinations. [b]. n.d.= not determined. HIV-1 RT = HIV-1 reverse transcriptase; CCD-19Lu = human lung fibroblast cells.

The HIV-1 RT inhibitory activity of Complex **1j** was found to be dose-dependent, % inhibition (concentration): 95% (50 μM), 76% (10 μM), 67% (5 μM), 58% (1 μM), 53% (0.5 μM) and 38% (0.1 μM). The $IC_{50}$ value was evaluated to be 0.31 ± 0.05μ M (Table 8 entry 1). The anti-HIV RT activity of Complex **1k** was also examined by the ELISA method, and an $IC_{50}$ value (0.57 ± 0.09 μM) was obtained. For comparison, it is noted that the free-base porphyrins (*i.e.*, $[H_2TMPyP]^4$ and $[H_2TPPS]^{4-}$) exhibited substantially lower HIV-1 RT inhibition activities than did the corresponding metal complex analogs, Complexes **1j** and **1k**, respectively. Likewise, the results in Table 8 show that Complexes **1a, 2a** and **3c** are also effective inhibitors of HIV-1 RT, with their measured $IC_{50}$ values being 28.4 ± 1.8 μM (**1a**), 0.33 ± 0.03 μM (**2a**) and 0.47 ± 0.09 μM (**3c**).

The Zn complex $[Zn^{II}(PP)]$ ($H_2PP$ = protoporphyrin) is reported to inhibit HIV-1 RT activity *in vitro (see* Argyris et al., J. Biol. Chem. 274:1549 (1991)), with a measured $IC_{50}$ value (ELISA assay) of 4.98 ± 0.93 μM. Based on the $IC_{50}$ values shown in Table 8, $[Zn^{II}(PP)]$ is at least 10-fold less potent for HIV-1 RT inhibition than the Gold(III) Complexes **1j , 1k, 2a** and **3c**.

FIG. 14 depicts the results of a comparative study for a series of gold complexes. By fixing the drug concentration at 6 μM, the HIV-1 RT inhibitory activities of the compounds were evaluated by the ELISA method. Up to about 70% HIV-1 RT inhibition was observed for Complexes **1j-k** and **2a**, whereas Complex **3c** was found to affect 64% RT inhibition under identical conditions. The data in FIG. 14 show that the presence of the gold atom is critical for the observed anti-HIV-1 RT activities, since all the free-base ligands are largely inactive (<30%) for RT inhibition. The results in FIG. 14

also show that $KAu^{III}Cl_4$ was found to result in 48% inhibition of the HIV-1 RT activities, compared to 70% inhibition attained by Complexes **1j**, **1k**, **2a** and **3c.** The result indicates that the chelating σ-donor ligands strongly influences the anti-HIV RT activities of the Gold(III) Complexes. Moreover, compared to gold(I) complex $[(Ph_3P)Au^{I}Cl]$, Complexes **1j**, **1k**, **2a** and **3c** are more powerful HIV-RT inhibitors *in vitro,* since treating the enzyme with $[(Ph_3P)Au^{I}Cl]$ (at 6 μM level) produced only 43% inhibition. As noted earlier, $[Zn^{II}(PP)]$ is a less effective HIV-1 RT inhibitor (compare 53% RT inhibition for $[Zn^{II}(PP)]$ to 70% RT inhibition for Complexes **1j**, **1k**, **2a** and **3c** under the same conditions). Likewise, the activity of the Zn complex $[Zn^{II}(TMPyP)]Cl_4$ was found to produce only 32% RT inhibition compared to, *e.g.*, about 70% for Complex **1j** at 6 μM level. The results indicate that the gold(III) atom plays a critical role in the RT inhibition activity.

Acute cytotoxicities of the gold(III) complexes to normal cells were evaluated using human lung fibroblast cells (CCD-19Lu) as model cell line. The percentages of cell survival at various doses of the gold complexes were determined by the MTT assay, and the toxicities profiles are shown in FIG. 15. The results suggest that Complexes **1j** and **1k** do not exhibit significant acute cytotoxicities to the fibroblast cells, with >90% cell survival being registered at drug concentration up to 96 μM, which is 16-fold of their $IC_{70}$ values (about 6 μM) for HIV-1 RT inhibition. However, 10% cell survival was observed when treating the fibroblast cells with Complexes **2a** and **3c** at 96 μM level. At 2 μM, Complex **1a** produced severe cytotoxicity, with 90% cell death being observed (*c.f.* $IC_{50}$ = 28 μM for HIV-1 RT inhibition).

Complex **1j** was chosen for further examination based on its solution stability and acute cytotoxicity profile. The HIV-1 RT inhibitory activities of Complex **1j** alone and in combination with AZT are shown in Figure 16. The results show that only 17 - 39% enzyme inhibition was achieved when treating HIV-1 RT with Complex **1j** (10, 50 and 100 nM) or AZT (8 nM) alone. However, when Complex **1j** was used in combination with AZT, a significant additive effect on HIV-1 RT inhibition was observed. Up to 68% enzyme inhibition was attained by employing the "Complex **1j** (100 nM) + AZT (8 nM)" combination. The $IC_{70}$ results show that the combination of Complex **1j** with AZT can lower the dosage requirement of the gold(III) porphyrin by 60-fold compared to using Complex **1j** alone. Without being limited by theory, it is believed that the synergistic HIV-1 RT inhibition by Complex **1j** and AZT results from the gold(III) porphyrin binding to certain sites nearby the active site of the enzyme. For example, $[Zn^{II}(PP)]$-mediated HIV-1 RT inhibition is reported to be related to binding of the metalloporphyrin to the connection domain sequence 398-407 (WETWWTEYWQ) (*see* Argyris et al., J. Biol. Chem. 274:1549 (1991)). In this work, the UV-vis absorption titration study revealed that Complex **1j** binds strongly to the WETWWTEYWQ sequence in aqueous buffer medium. Addition of the peptide (0- 5 μM) to Complex **1j** produced isosbestic spectral changes (isosbestic points at 314, 417, 508 and 532 nm) with significant hypochromicity (58 %) and small bathochromic shift (7 nm) of the Soret band. Using the absorbance data of the Soret band, the linear plot ($R$ = 0.99) of [peptide] / $\Delta\varepsilon_{ap}$ *vs* [peptide] gave the intrinsic binding constant ($K_b$) = (7.9 ± 0.7) x $10^4$ $dm^3mol^{-1}$ at 292 *K*. A comparable $K_b$ value [(1.3 ± 0.1) x $10^5$ $dm^3$ $mol^{-1}$ at 292 *K*] was obtained for the $[Zn^{II}(PP)]$-WETWWTEYWQ interaction based on UV-vis absorption titration study. No significant binding of Complex **1k** with the peptide was observed based on UV-vis spectral titration. Given that Complex **1k** is also an effective HIV-1 RT inhibitor, the poor binding affinity to WETWWTEYWQ may suggest an alternative binding site for the complex.

## Claims

1. A composition for use in the treatment of tumor or HIV, comprising an effective amount of a gold (III) complex of formula:

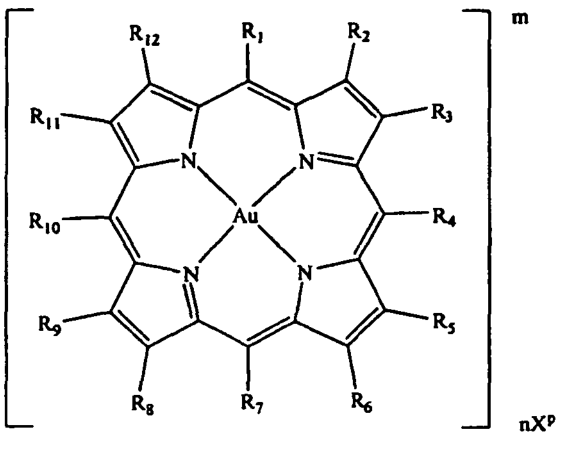

or a pharmaceutically acceptable salt thereof, and a pharmaceutically acceptable carrier, wherein:

(a) $R_1$, $R_4$, $R_7$ and $R_{10}$ are each -phenyl, -4-methylphenyl, -4-methoxyphenyl, -4-bromophenyl, -4-chlorophenyl, -3,4,5-trimethoxyphenyl or -3,4,5-trifluorophenyl, and
$R_2$, $R_3$, $R_5$, $R_6$, $R_8$, $R_9$, $R_{11}$ and $R_{12}$ are each -H;
$X^P$ is $Cl^-$; m is 1; and n is 1; or
(b) $R_1$, $R_4$, $R_7$ and $R_{10}$ are each-4-sulfonatophenyl;
$R_2$, $R_3$, $R_5$, $R_6$, $R_8$, $R_9$, $R_{11}$ and $R_{12}$ are each -H;
$X^P$ is $Na^+$; m is +3; and n is 3; or
(c) $R_1$, $R_4$, $R_7$ and $R_{10}$ are each -H, and
$R_2$, $R_3$, $R_5$, $R_6$, $R_8$, $R_9$, $R_{11}$ and $R_{12}$ are each -ethyl, or $R_2$ and $R_{11}$ are each - ethyl, $R_3$, $R_5$, $R_9$ and $R_{12}$ are each -methyl and $R_6$ and $R_8$ are each -methyl-3-propanoate;
$X^P$ is $cl^-$; m is 1; and n is 1.

**2.** A composition for use in the treatment of tumor or HIV, comprising an effective amount of a gold (III) complex of formula:

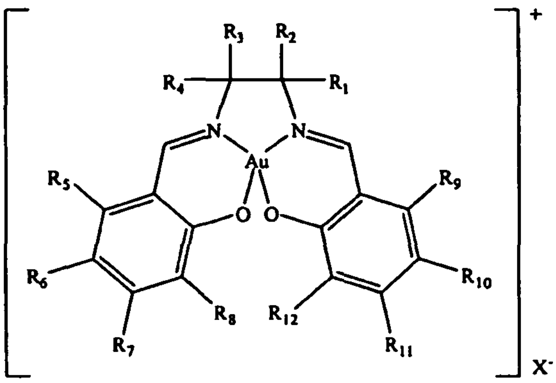

or a pharmaceutically acceptable salt thereof, and a pharmaceutically acceptable carrier, wherein:

$R_1$- $R_{12}$ are each independently -H, -halo, -($C_1$-$C_6$)alkyl or -O($C_1$-$C_6$)alkyl which may be substituted with one or more -O($C_1$-$C_6$)alkyl or-halo; and
X is a counter-anion.

**3.** The composition for use according to claim 2, wherein $R_1$-$R_4$ are each -H; and X is $Cl^-$.

**4.** The composition for use according to claim 3, wherein $R_5$-$R_{12}$ are each -H.

**5.** The composition for use according to claim 3, wherein $R_5$, $R_7$-$R_9$ and $R_{11}$-$R_{12}$ are each -H; and $R_6$ and $R_{10}$ are each -Cl.

**6.** The composition for use according to claim 3, wherein $R_5$, $R_7$, $R_9$ and $R_{10}$ are each -H; and $R_6$, $R_8$, $R_{10}$ and $R_{12}$ are each -Cl.

**7.** A composition for use in the treatment of tumor or HIV, comprising an effective amount of a gold (III) complex of formula:

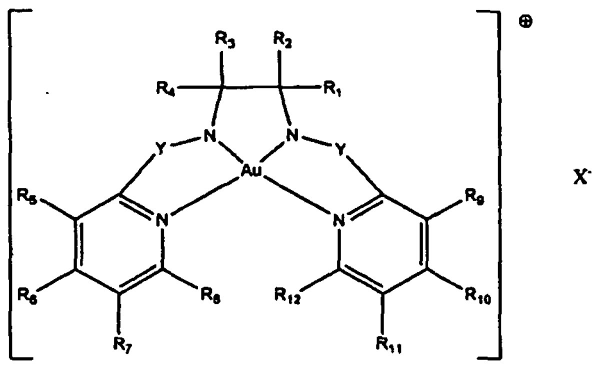

or a pharmaceutically acceptable salt thereof, and a pharmaceutically acceptable carrier, wherein:

(a) $R_1$- $R_{12}$ are each independently -H, -halo, -($C_1$-$C_6$)alkyl or -O($C_1$-$C_6$)alkyl which may be substituted with one or more -O($C_1$-$C_6$)alkyl or-halo; or
(b) $R_1$ and $R_4$ are absent, and $R_2$ and $R_3$ together form a 6-membered aryl ring of formula:

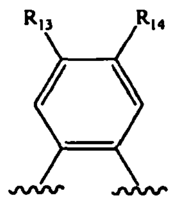

Y is CO or $SO_2$;
$R_{13}$ and $R_{14}$ are each-H or-halo; and
X is a counter-anion.

**8.** The composition for use according to claim 7, wherein Y is CO; and X is $Cl^-$.

**9.** The composition for use according to claim 8, wherein $R_1$-$R_{12}$ are each -H.

**10.** The composition for use according to claim 8, wherein $R_1$-$R_4$ are each -methyl; and $R_5$-$R_{12}$ are each- H.

**11.** The composition for use according to claim 8, wherein $R_1$ and $R_4$-$R_{12}$ are each -H; and $R_2$ and $R_3$ are each -phenyl.

**12.** The composition for use according to claim 8, wherein $R_1$ and $R_4$ are absent; $R_2$ and $R_3$ together form

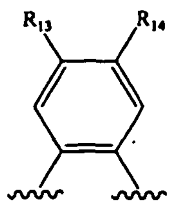 ;

and
$R_5$-$R_{12}$ are each -H.

**13.** A pharmaceutical composition comprising an effective amount of a gold (III) complex of formula:

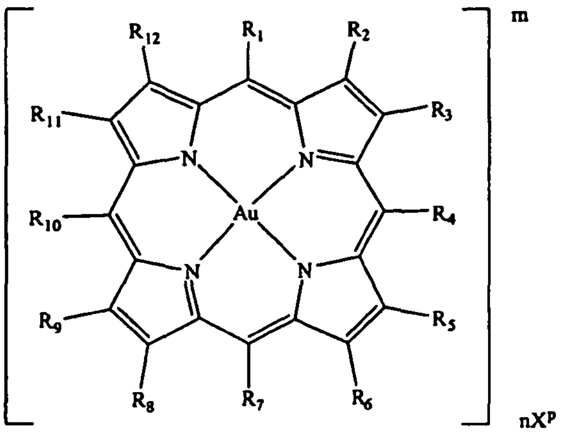

or a pharmaceutically acceptable salt thereof, and a pharmaceutically acceptable carrier, wherein:

(a) $R_1$, $R_4$, $R_7$ and $R_{10}$ are each-phenyl, -4-methylphenyl, -4-methoxyphenyl, -4-bromophenyl, -4-chlorophenyl, -3,4,5-trimethoxyphenyl or -3,4,5-trifluorophenyl, and
$R_2$, $R_3$, $R_5$, $R_6$, $R_8$, $R_9$, $R_{11}$ and $R_{12}$ are each -H;
$X^p$ is $Cl^-$; m is 1; and n is l; or
(b) $R_1$, $R_4$, $R_7$ and $R_{10}$ are each-4-sulfonatophenyl ;
$R_2$, $R_3$, $R_5$, $R_6$, $R_8$, $R_9$, $R_{11}$ and $R_{12}$ are each -H;
$X^p$ is $Na^+$; m is +3; and n is 3; or
(c) $R_1$, $R_4$, $R_7$ and $R_{10}$ are each -H, and
$R_2$, $R_3$, $R_5$, $R_6$, $R_8$, $R_9$, $R_{11}$ and $R_{12}$ are each -ethyl, or $R_2$ and $R_{11}$ are each - ethyl, $R_3$, $R_5$, $R_9$ and $R_{12}$ are each -methyl and $R_6$ and $R_8$ are each -methyl-3-propanoate;
$X^p$ is $Cl^-$; m is 1; and n is 1.

**14.** A pharmaceutical composition comprising an effective amount of a gold (III) complex of formula:

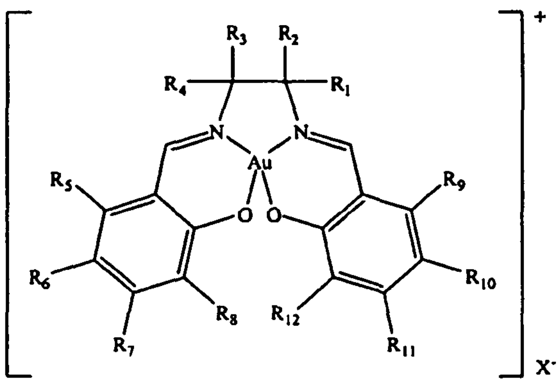

or a pharmaceutically acceptable salt thereof, and a pharmaceutically acceptable carrier, wherein:

$R_1$- $R_{12}$ are each independently -H, -halo, -($C_1$-$C_6$)alkyl or -O($C_1$-$C_6$)alkyl which may be substituted with one or more -O($C_1$-$C_6$)alkyl or-halo; and
X is a counter-anion.

**15.** A pharmaceutical composition comprising an effective amount of a gold (III) complex of formula:

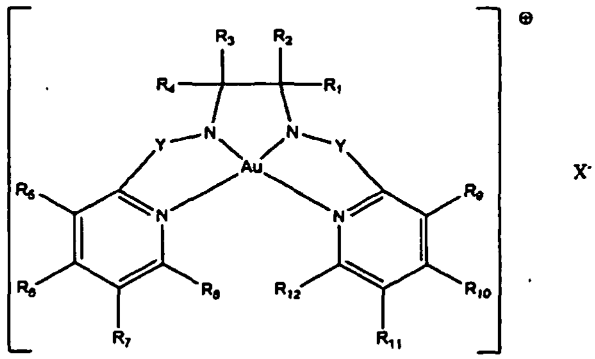

or a pharmaceutically acceptable salt thereof, and a pharmaceutically acceptable carrier, wherein:

(a) $R_1$- $R_{12}$ are each independently -H, -halo, -($C_1$-$C_6$)alkyl or -O($C_1$-$C_6$)alkyl which may be substituted with one or more -O($C_1$-$C_6$)alkyl or-halo; and X is a counter-anion, or
(b) $R_1$ and $R_4$ are absent; and $R_2$ and $R_3$ together form a 6-membered aryl ring of formula:

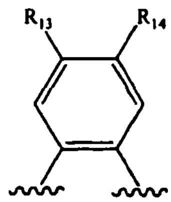

Y is CO or $SO_2$;
$R_{13}$ and $R_{14}$ are each-H or-halo; and
X is a counter-anion.

**16.** The composition of claim 14 or claim 15 further comprising AZT.

**17.** The composition according to claim 16 for use in the treatment of HIV,
wherein the composition comprises an effective amount of the gold (III) complex of claim 14 or claim 15.

## Patentansprüche

**1.** Zusammensetzung zur Verwendung bei der Behandlung von Tumor oder HIV, umfassend eine wirksame Menge eines Gold (III)-Komplexes der Formel:

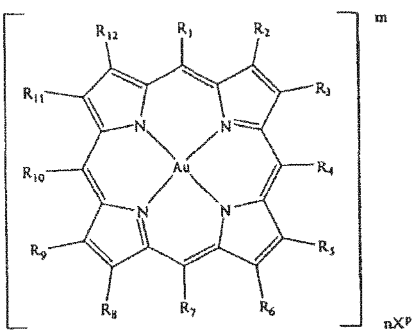

oder ein pharmazeutisch verträgliches Salz davon und einen pharmazeutisch verträglichen Träger, worin:

(a) $R_1$, $R_4$, $R_7$ und $R_{10}$ jeweils -phenyl, -4-methylphenyl, -4-methoxyphenyl, -4-bromphenyl, -4-chlorphenyl, -3,4,5-trimethoxyphenyl oder -3,4,5-trifiluorphenyl sind, und

$R_2$, $R_3$, $R_5$, $R_6$, $R_8$, $R_9$, $R_{11}$ und $R_{12}$ jeweils -H sind;
$X^p$ $Cl^-$ ist, m 1 ist; und n 1 ist; oder
(b) $R_1$, $R_4$, $R_7$ und $R_{10}$ jeweils -4-sulfonatophenyl sind;
$R_2$, $R_3$, $R_5$, $R_6$, $R_8$, $R_9$, $R_{11}$ und $R_{12}$ jeweils -H sind;
$X^p$ $Na^+$ ist, m +3 ist; und n 3 ist; oder
(c) $R_1$, $R_4$, $R_7$ und $R_{10}$ jeweils H sind; und
$R_2$, $R_3$, $R_5$, $R_6$, $R_8$, $R_9$, $R_{11}$ und $R_{12}$ jeweils -ethyl sind; oder $R_2$ und $R_{11}$ jeweils -ethyl sind, $R_3$, $R_5$, $R_9$ und $R_{12}$ jeweils -methyl sind und $R_6$ und $R_8$ jeweils -methyl-3-propanoat sind;
$X^p$ $Cl^-$ ist; m 1 ist; und n 1 ist.

**2.** Zusammensetzung zur Verwendung bei der Behandlung von Tumor oder HIV, umfassend eine wirksame Menge eines Gold (III)-Komplexes der Formel:

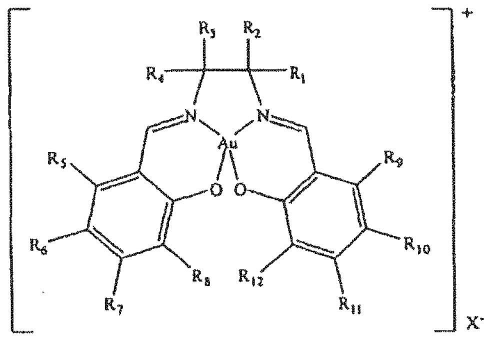

oder ein pharmazeutisch verträgliches Salz davon und einen pharmazeutisch verträglichen Träger, worin:

$R_1$-$R_{12}$ unabhängig -H, -halogen, -($C_1$-$C_6$)Alkyl oder -O($C_1$-$C_6$)Alkyl sind, welche substituiert sein können mit einem oder mehreren aus -O($C_1$-$C_6$)Alkyl oder -halogen; und X ein Gegenanion ist.

**3.** Zusammensetzung zur Verwendung nach Anspruch 2, worin $R_1$-$R_4$ jeweils -H sind; und X $Cl^-$ ist.

**4.** Zusammensetzung zur Verwendung nach Anspruch 3, worin $R_5$-$R_{12}$ jeweils -H sind.

**5.** Zusammensetzung zur Verwendung nach Anspruch 3, worin $R_5$, $R_7$-$R_9$ und $R_{11}$-$R_{12}$ jeweils -H sind; und $R_6$ und $R_{10}$ jeweils -Cl sind.

**6.** Zusammensetzung zur Verwendung nach Anspruch 3, worin $R_5$, $R_7$, $R_9$ und $R_{10}$ jeweils -H sind; und $R_6$, $R_8$, $R_{10}$ und $R_{12}$ jeweils -Cl sind.

**7.** Zusammensetzung zur Verwendung bei der Behandlung von Tumor oder HIV, umfassend eine wirksame Menge eines Gold (III)-Komplexes der Formel:

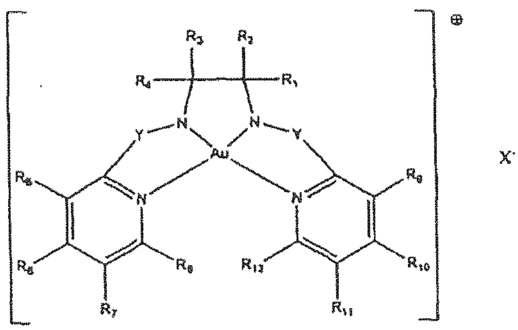

oder ein pharmazeutisch verträgliches Salz davon und einen pharmazeutisch verträglichen Träger, worin:

(a) $R_1$-$R_{12}$ unabhängig -H, -halogen, -($C_1$-$C_6$)Alkyl oder -O($C_1$-$C_6$)Alkyl sind, welche substituiert sein können mit einem oder mehreren aus -O($C_1$-$C_6$)Alkyl oder-halogenr oder
(b) $R_1$ und $R_4$ nicht vorliegen und $R_2$ und $R_3$ zusammen einen 6-gliedrigen Arylring der Formel:

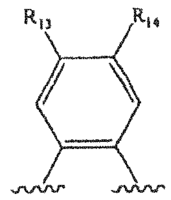

bildern,
Y CO oder $SO_2$ ist;
$R_{13}$ und $R_{14}$ jeweils -H oder -halogen sind;
und X ein Gegenanion ist.

**8.** Zusammensetzung zur Verwendung nach Anspruch 7, worin Y CO ist; und X $Cl^-$ ist.

**9.** Zusammensetzung zur Verwendung nach Anspruch 8, worin $R_1$-$R_{12}$ jeweils -H sind.

**10.** Zusammensetzung zur Verwendung nach Anspruch 8, worin $R_1$-$R_4$ jeweils -methyl sind; und $R_5$-$R_{12}$ jeweils -H sind.

**11.** Zusammensetzung zur Verwendung nach Anspruch 8, worin $R_1$ und $R_4$-$R_{12}$ jeweils -H sind; und $R_3$ und $R_3$ jeweils Phenyl sind.

**12.** Zusammensetzung zur Verwendung nach Anspruch 8, worin $R_1$ und $R_4$ nicht vorliegen; $R_2$ und $R_3$ zusammen

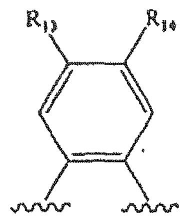

bilden; und
$R_5$-$R_{12}$ jeweils -H sind.

**13.** Pharmazeutische Zusammensetzung, umfassend eine wirksame Menge eines Gold (III)-komplexes der Formel:

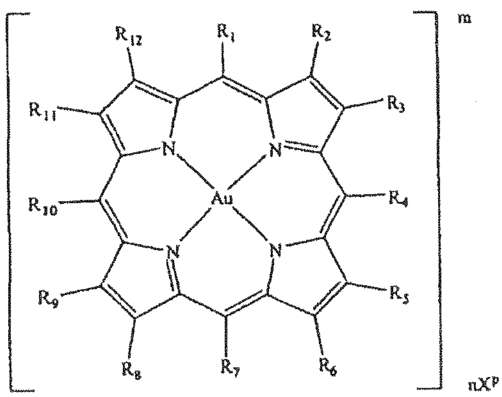

oder ein pharmazeutisch verträgliches Salz davon und einen pharmazeutisch verträglichen Träger, worin:

(a) $R_1$, $R_4$, $R_7$ und $R_{10}$ jeweils -phenyl, -4-methylphenyl, -4-methoxyphenyl, -4-bromphenyl, -4-chlorphenyl, -3,4,5-trimethoxyphenyl oder -3,4,5-trifluorphenyl sind, und
$R_2$, $R_3$, $R_5$, $R_6$, $R_8$, $R_9$, $R_{11}$ und $R_{12}$ jeweils -H sind;
$X^p$ $Cl^-$ ist; m 1 ist; und n 1 ist; oder
(b) $R_1$, $R_4$, $R_7$ und $R_{10}$ jeweils -4-sulfonatophenyl sind;
$R_2$, $R_3$, $R_5$, $R_6$, $R_8$, $R_9$, $R_{11}$ und $R_{12}$ jeweils -H sind;
$X^p$ $Na^+$ ist, m +3 ist; und n 3 ist; oder
(c) $R_1$, $R_4$, $R_7$ und $R_{10}$ jeweils H sind; und
$R_2$, $R_3$, $R_5$, $R_6$, $R_8$, $R_9$, $R_{11}$ und $R_{12}$ jeweils -ethyl sind; oder $R_2$ und $R_{11}$ jeweils -ethyl sind, $R_3$, $R_5$, $R_9$ und $R_{12}$ jeweils -methyl sind und $R_6$ und $R_8$ jeweils -methyl-3-propanoat sind;
$X^P$ $Cl^-$ ist; m 1 ist; und n 1 ist.

**14.** Pharmazeutische Zusammensetzung, umfassend eine wirksame Menge eines Gold (III)-Komplexes der Formel:

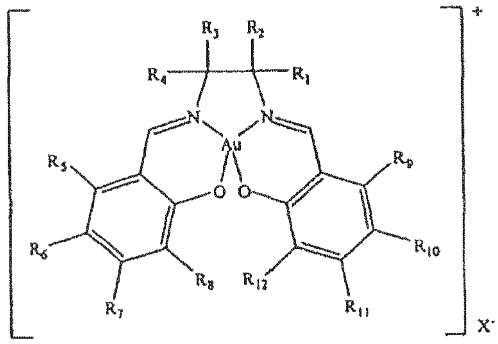

oder ein pharmazeutisch verträgliches Salz davon und einen pharmazeutisch verträglichen Träger, worin:

$R_1$-$R_{12}$ unabhängig -H, -halogen, -($C_1$-$C_6$)Alkyl oder -O($C_1$-$C_6$)Alkyl sind, welche substituiert sein können mit einem oder mehreren aus -O($C_1$-$C_6$)Alkyl oder -halogen; und X ein Gegenanion ist.

**15.** Pharmazeutische Zusammensetzung, umfassend eine wirksame Menge eines Gold (III)-Komplexes der Formel:

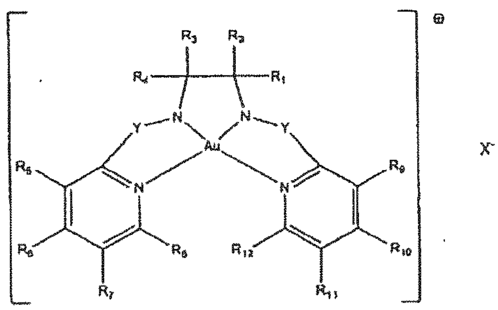

oder ein pharmazeutisch verträgliches Salz davon und einen pharmazeutisch verträglichen Träger, worin:

(a) $R_1$-$R_{12}$ unabhängig -H, -halogen, -($C_1$-$C_6$)Alkyl oder -O($C_1$-$C_6$)Alkyl sind, welche substituiert sein können mit einem oder mehreren aus -O($C_1$-$C_6$)Alkyl oder -halogen; und X ein Gegenanion ist; oder
(b) $R_1$ und $R_4$ nicht vorliegen und $R_2$ und $R_3$ zusammen einen 6-gliedrigen Arylring der Formel:

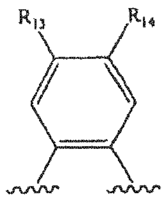

bilden,
Y CO oder $SO_2$ ist;
$R_{13}$ und $R_{14}$ jeweils -H oder -halogen sind;
und X ein Gegenanion ist.

16. Zusammensetzung nach Anspruch 14 oder Anspruch 15, weiterhin umfassend AZT.

17. Zusammensetzung nach Anspruch 16 zur Verwendung bei der Behandlung von HIV, worin die Zusammensetzung eine wirksame Menge des Gold (III)-Komplexes nach Anspruch 14 oder Anspruch 15 umfasst.

## Revendications

1. Composition pour utilisation dans le traitement d'une tumeur ou du VIH, comprenant une quantité efficace d'un complexe d'or(III) de formule :

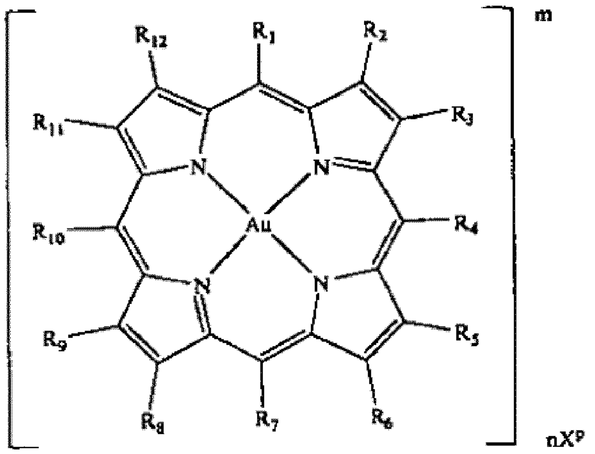

ou d'un sel pharmaceutiquement acceptable de celui-ci, et un véhicule pharmaceutiquement acceptable, formule dans laquelle :

(a) chacun de $R_1$, $R_4$, $R_7$ et $R_{10}$ est -phényle, -4-méthyl-phényle, -4-méthoxyphényle, -4-bromophényle, -4-chlorophényle, -3,4,5-triméthoxyphényle ou -3,4,5-trifluorophényle, et
chacun de $R_2$, $R_3$, $R_5$, $R_6$, $R_8$, $R_9$, $R_{11}$ et $R_{12}$ est -H ;
$X^p$ est $Cl^-$ ; m vaut 1 ; et n vaut 1 ; ou
(b) chacun de $R_1$, $R_4$, $R_7$ et $R_{10}$ est -4-sulfonatophényle ;
chacun de $R_2$, $R_3$, $R_5$, $R_6$, $R_8$, $R_9$, $R_{11}$ et $R_{12}$ est -H ;
$X^P$ est $Na^+$ ; m vaut +3 ; et n vaut 3 ; ou
(c) chacun de $R_1$, $R_4$, $R_7$ et $R_{10}$ est -H ; et
chacun de $R_2$, $R_3$, $R_5$, $R_6$, $R_8$, $R_9$, $R_{11}$ et $R_{12}$ est -éthyle, ou bien chacun de $R_2$ et $R_{11}$ est -éthyle, chacun de $R_3$, $R_5$, $R_9$ et $R_{12}$ est -méthyle et chacun de $R_6$ et $R_8$ est -méthyl-3-propanoate ;
$X^p$ est $Cl^-$ ; m vaut 1 ; et n vaut 1.

2. Composition pour utilisation dans le traitement d'une tumeur ou du VIH, comprenant une quantité efficace d'un complexe d'or(III) de formule :

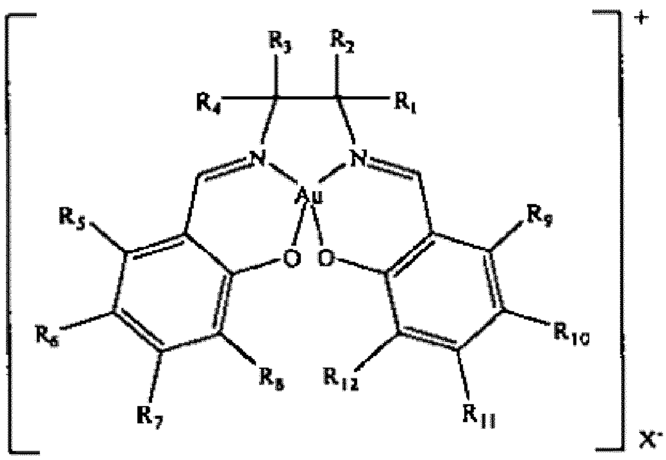

ou d'un sel pharmaceutiquement acceptable de celui-ci, et un véhicule pharmaceutiquement acceptable, formule dans laquelle :

chacun de $R_1$ à $R_{12}$ est indépendamment -H, -halogéno, -alkyle en $C_1$ à $C_6$ ou -O-alkyle en $C_1$ à $C_6$ qui peut être substitué par un ou plusieurs -O-alkyle en $C_1$ à $C_6$ ou -halogéno ; et
X est un contre-anion.

**3.** Composition pour utilisation selon la revendication 2, dans laquelle chacun de $R_1$ à $R_4$ est -H ; et X est $Cl^-$.

**4.** Composition pour utilisation selon la revendication 3, dans laquelle chacun de $R_5$ à $C_{12}$ est -H.

**5.** Composition pour utilisation selon la revendication 3, dans laquelle chacun de $R_5$, $R_7$ à $R_9$ et $R_{11}$ et $R_{12}$ est -H ; et chacun de $R_6$ et $R_{10}$ est -Cl.

**6.** Composition pour utilisation selon la revendication 3, dans laquelle chacun de $R_5$, $R_7$, $R_9$ et $R_{10}$ est -H ; et chacun de $R_6$, $R_8$, $R_{10}$ et $R_{12}$ est -Cl.

**7.** Composition pour utilisation dans le traitement d'une tumeur ou du VIH, comprenant une quantité efficace d'un complexe d'or(III) de formule :

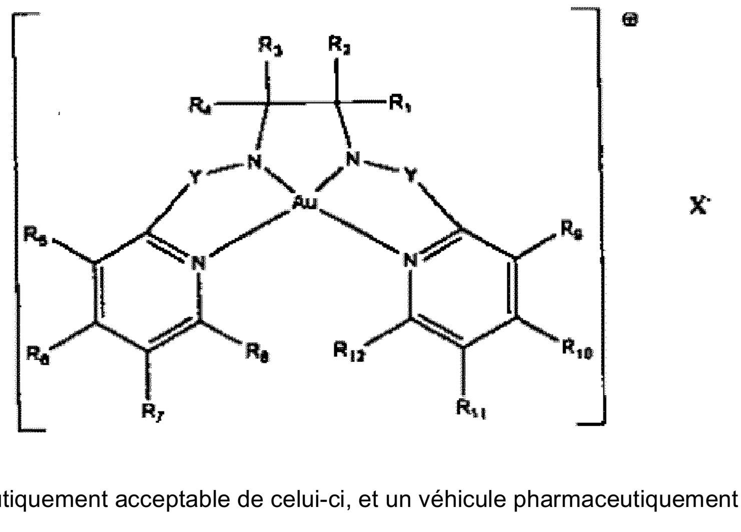

ou d'un sel pharmaceutiquement acceptable de celui-ci, et un véhicule pharmaceutiquement acceptable, formule dans laquelle :

(a) chacun de $R_1$ à $R_{12}$ est indépendamment -H, -halogéno, -alkyle en $C_1$ à $C_6$ ou -O-alkyle en $C_1$ à $C_6$ qui peut être substitué par un ou plusieurs -O-alkyle en $C_1$ à $C_6$ ou -halogéno ; ou
(b) $R_1$ et $R_4$ sont absents ; et $R_2$ et $R_3$ forment ensemble un cycle aryle à 6 chaînons de formule :

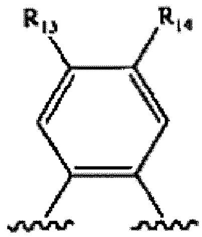

Y est CO ou $SO_2$ ;
chacun de $R_{13}$ et $R_{14}$ est -H ou -halogéno ; et
X est un contre-anion.

**8.** Composition pour utilisation selon la revendication 7, dans laquelle Y est CO ; et X est $Cl^-$.

**9.** Composition pour utilisation selon la revendication 8, dans laquelle chacun de $R_1$ à $R_{12}$ est -H.

**10.** Composition pour utilisation selon la revendication 8, dans laquelle chacun de $R_1$ à $R_4$ est -méthyle ; et chacun de $R_5$ à $R_{12}$ est -H.

**11.** Composition pour utilisation selon la revendication 8, dans laquelle chacun de $R_1$ et $R_4$ à $R_{12}$ est -H ; et chacun de $R_2$ et $R_3$ est -phényle.

**12.** Composition pour utilisation selon la revendication 8, dans laquelle $R_1$ et $R_4$ sont absents ; $R_2$ et $R_3$ forment ensemble

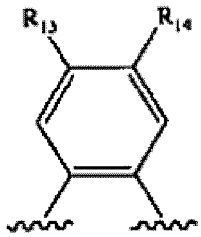

et chacun de $R_5$ à $R_{12}$ est -H.

**13.** Composition pharmaceutique comprenant une quantité efficace d'un complexe d'or(III) de formule :

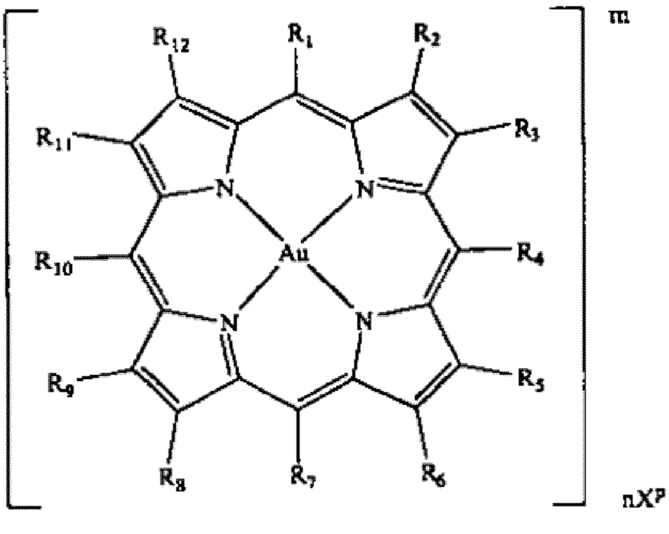

ou d'un sel pharmaceutiquement acceptable de celui-ci, et
un véhicule pharmaceutiquement acceptable,

formule dans laquelle :

(a) chacun de $R_1$, $R_4$, $R_7$ et $R_{10}$ est -phényle, -4-méthyl-phényle, -4-méthoxyphényle, -4-bromophényle, -4-chlorophényle, -3,4,5-triméthoxyphényle ou -3,4,5-trifluorophényle, et
chacun de $R_2$, $R_3$, $R_5$, $R_6$, $R_8$, $R_9$, $R_{11}$ et $R_{12}$ est -H ;
$X^p$ est $Cl^-$ ; m vaut 1 ; et n vaut 1 ; ou
(b) chacun de $R_1$, $R_4$, $R_7$ et $R_{10}$ est -4-sulfonatophényle ;
chacun de $R_2$, $R_3$, $R_5$, $R_6$, $R_8$, $R_9$, $R_{11}$ et $R_{12}$ est -H ;
$X^p$ est $Na^+$ ; m vaut +3 ; et n vaut 3 ; ou
(c) chacun de $R_1$, $R_4$, $R_7$ et $R_{10}$ est -H ; et
chacun de $R_2$, $R_3$, $R_5$, $R_6$, $R_8$, $R_9$, $R_{11}$ et $R_{12}$ est -éthyle, ou bien chacun de $R_2$ et $R_{11}$ est -éthyle, chacun de $R_3$, $R_5$, $R_9$ et $R_{12}$ est -méthyle et chacun de $R_6$ et $R_8$ est -méthyl-3-propanoate ;
$X^p$ est $Cl^-$ ; m vaut 1 ; et n vaut 1.

**14.** Composition pharmaceutique comprenant une quantité efficace d'un complexe d'or(III) de formule :

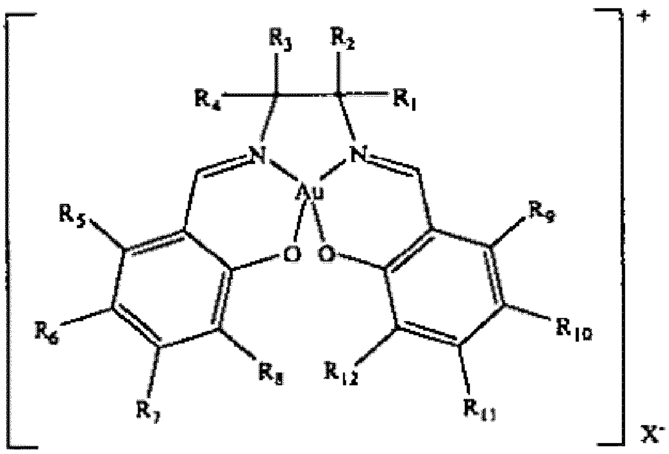

ou d'un sel pharmaceutiquement acceptable de celui-ci, et un véhicule pharmaceutiquement acceptable, formule dans laquelle :

chacun de $R_1$ à $R_{12}$ est indépendamment -H, -halogéno, -alkyle en $C_1$ à $C_6$ ou -O-alkyle en $C_1$ à $C_6$ qui peut être substitué par un ou plusieurs -O-alkyle en $C_1$ à $C_6$ ou- halogéno ; et
X est un contre-anion.

**15.** Composition pharmaceutique comprenant une quantité efficace d'un complexe d'or(III) de formule :

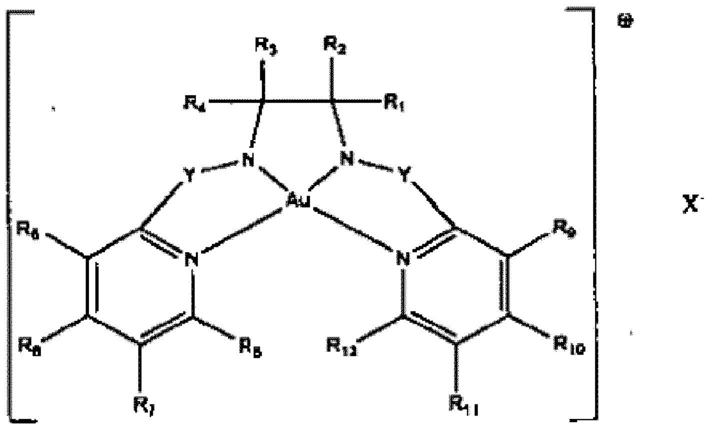

ou d'un sel pharmaceutiquement acceptable de celui-ci, et un véhicule pharmaceutiquement acceptable, formule dans laquelle :

(a) chacun de $R_1$ à $R_{12}$ est indépendamment -H, -halogéno, -alkyle en $C_1$ à $C_6$ ou -O-alkyle en $C_1$ à $C_6$ qui peut être substitué par un ou plusieurs -O-alkyle en $C_1$ à $C_6$ ou -halogéno ; et
X est un contre-anion, ou
(b) $R_1$ et $R_4$ sont absents ; et $R_2$ et $R_3$ forment ensemble un cycle aryle à 6 chaînons de formule :

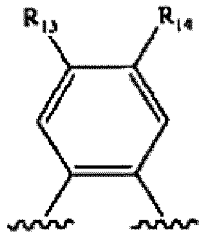

Y est CO ou $SO_2$ ;
chacun de $R_{13}$ et $R_{14}$ est -H ou -halogéno ; et
X est un contre-anion.

**16.** Composition selon la revendication 14 ou la revendication 15, comprenant en outre de l'AZT.

**17.** Composition selon la revendication 16, pour utilisation dans le traitement du VIH, laquelle composition comprend une quantité efficace du complexe d'or(III) de la revendication 14 ou de la revendication 15.

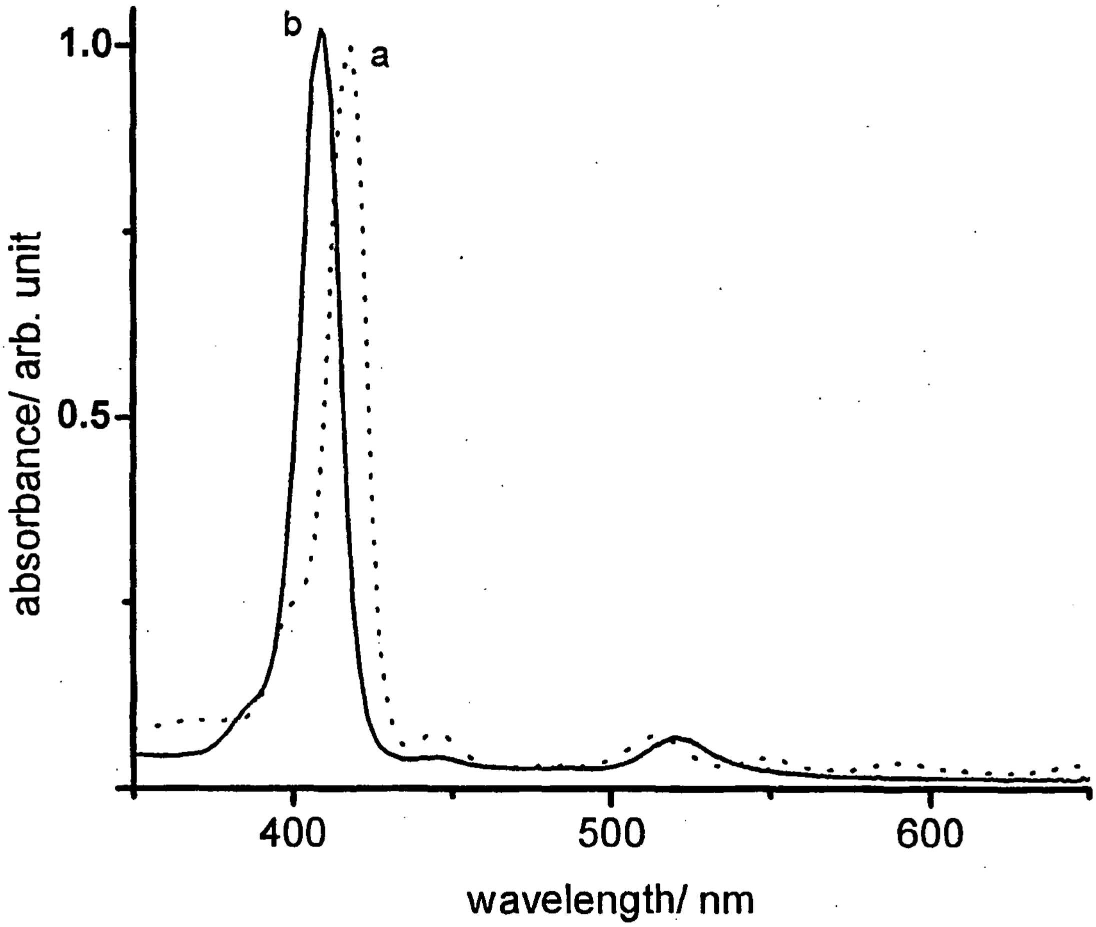


FIG. 1

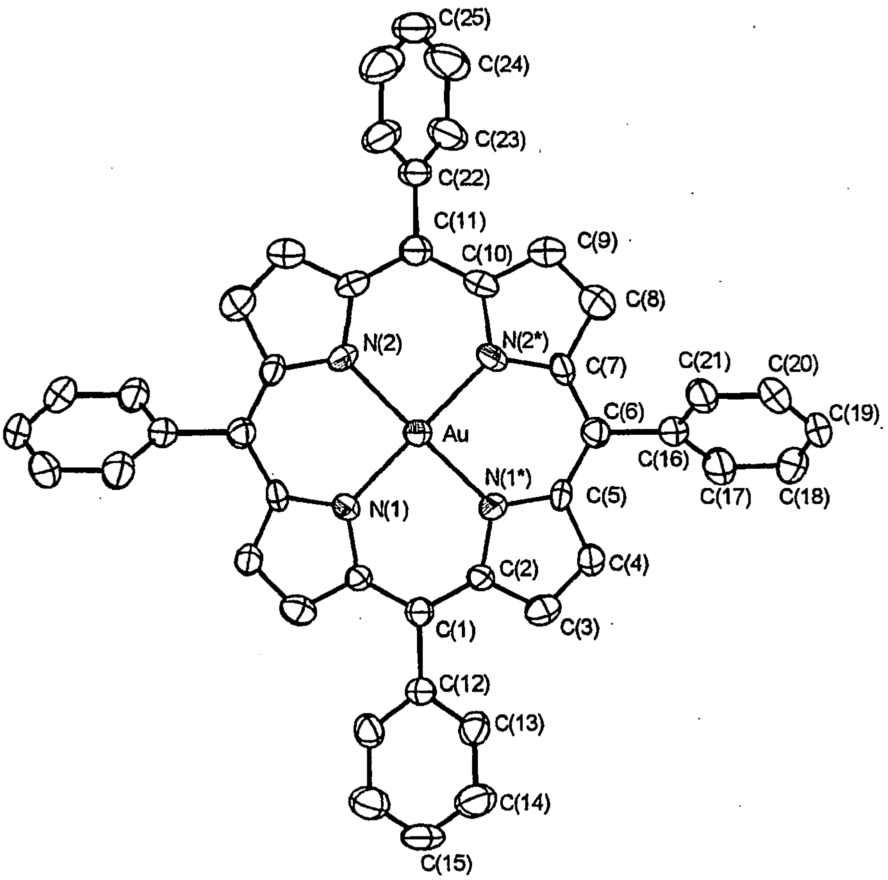

FIG. 2

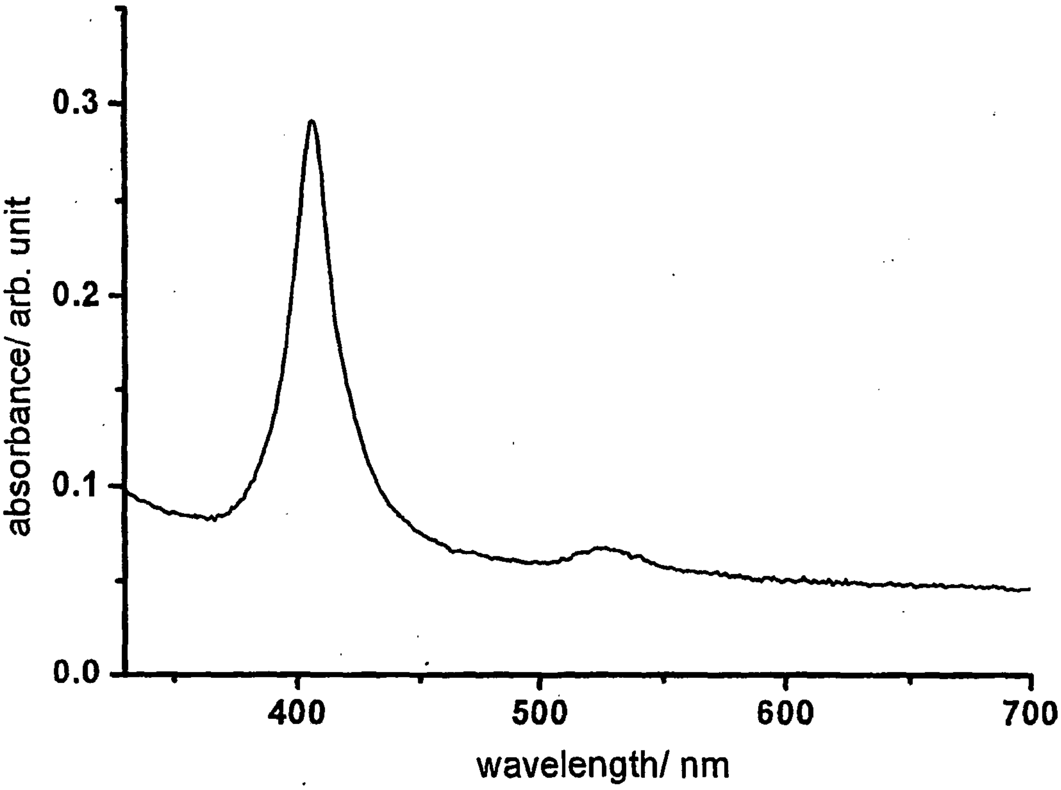


FIG. 3

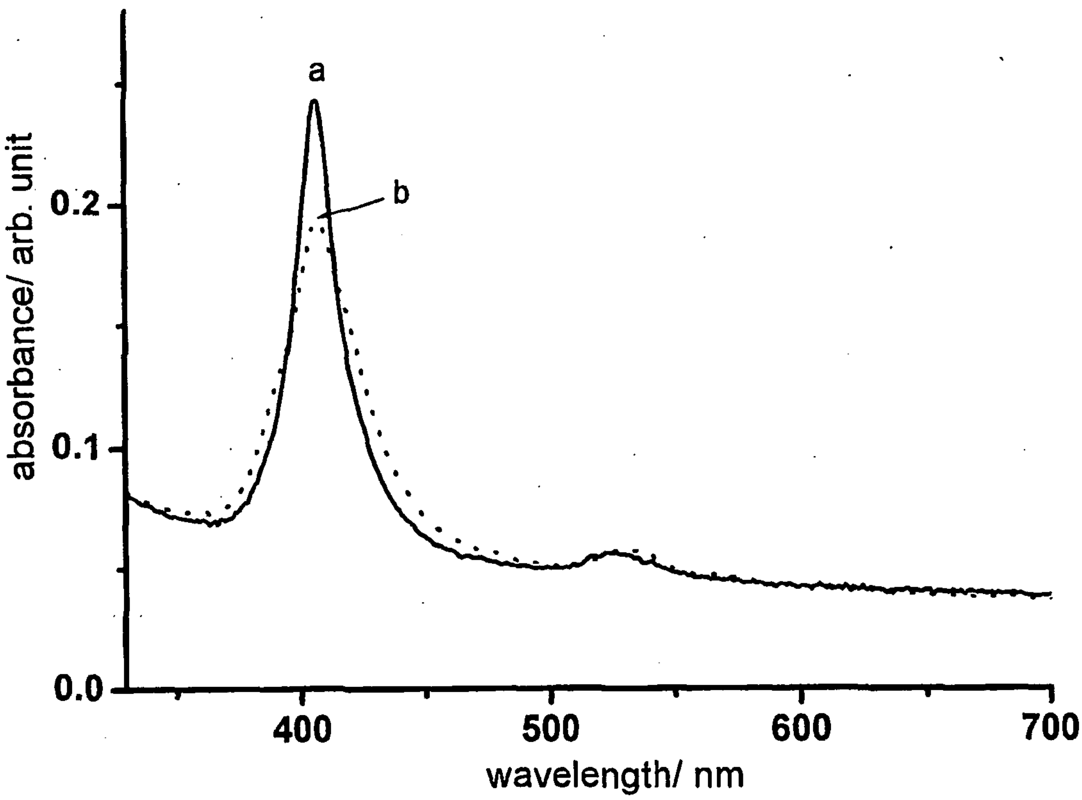


FIG. 4

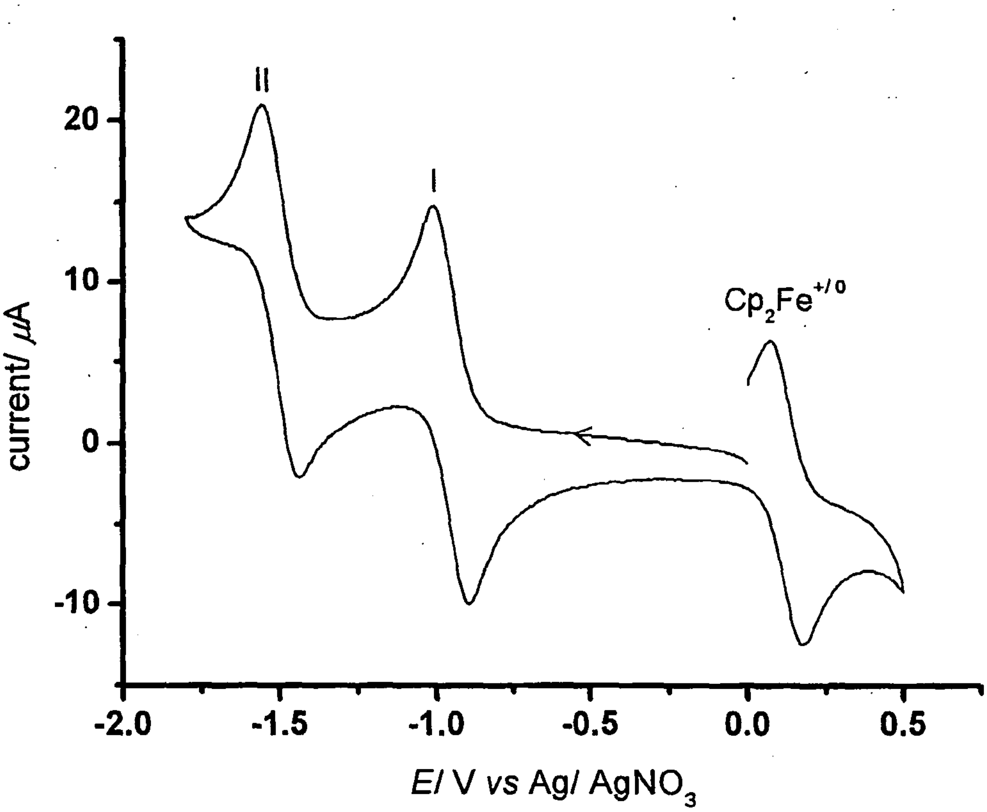


FIG. 5

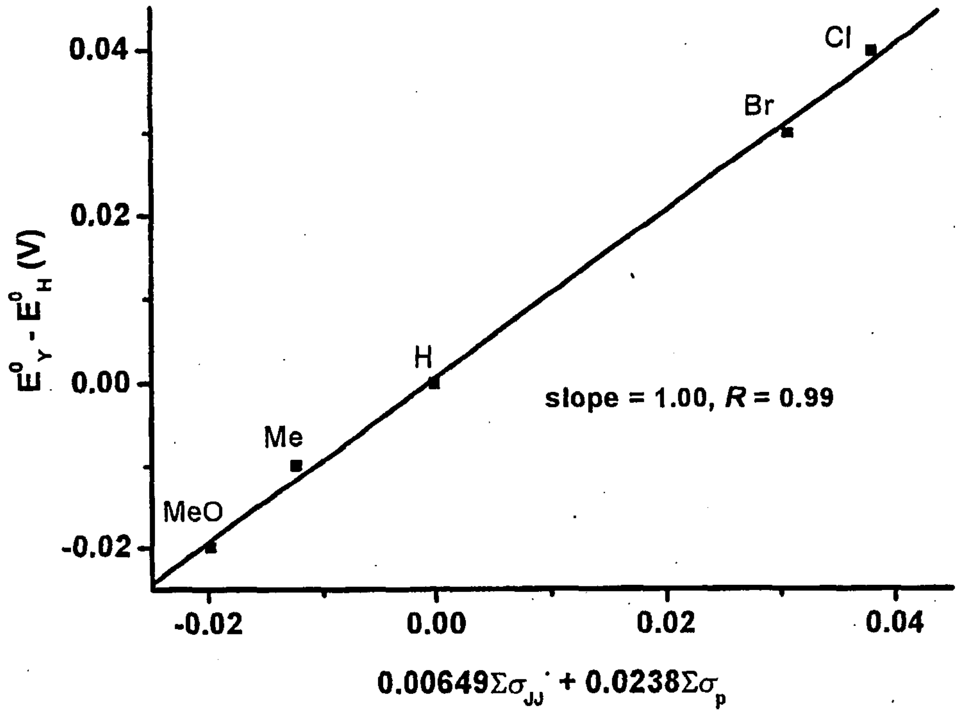
0.04
0.02
0.00
-0.02
E0Y - E0H (V)
Cl
Br
H
Me
MeO
slope = 1.00, R = 0.99
-0.02
0.00
0.02
0.04
0.00649Σσ JJ˙ + 0.0238Σσp

FIG. 6

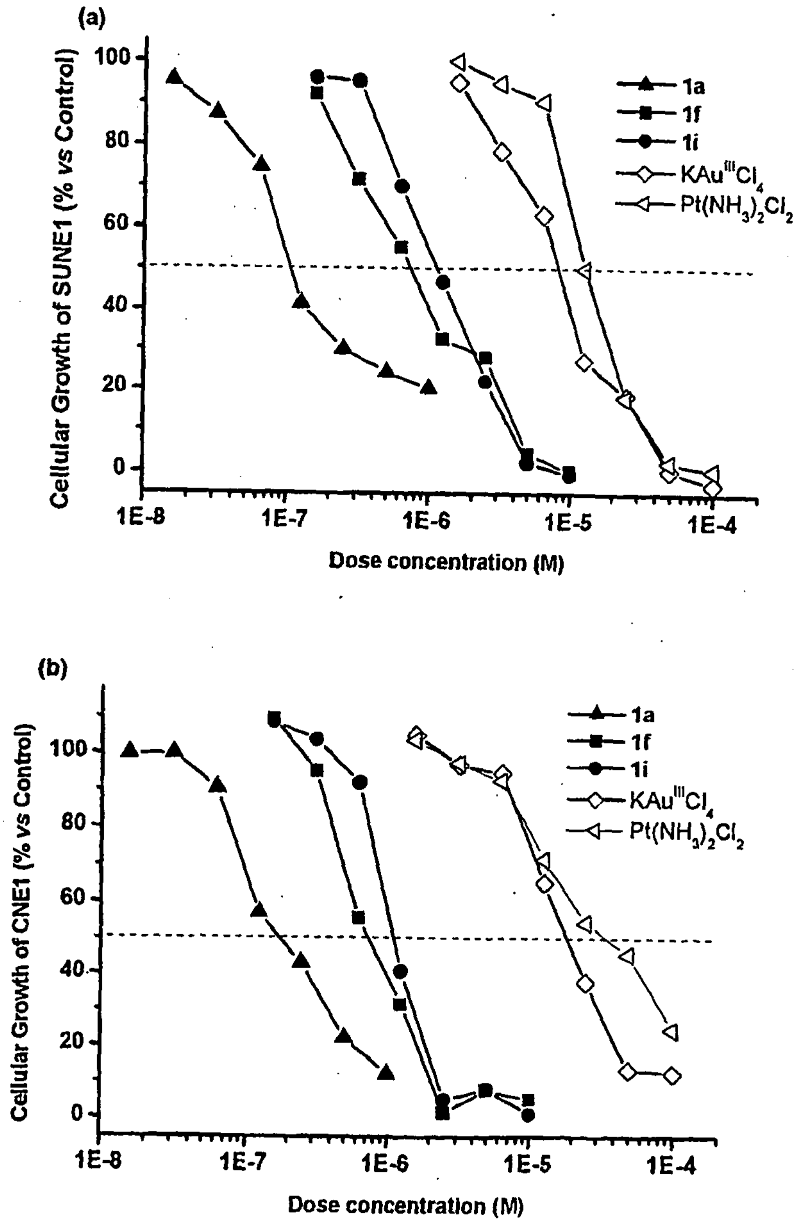
(a)
Cellular Growth of SUNE1 (% vs Control)
100
80
60
40
20
0
1E-8
1E-7
1E-6
1E-5
1E-4
Dose concentration (M)
1a
1f
1i
KAu$^{III}$Cl$_4$
Pt(NH$_3$)$_2$Cl$_2$
(b)
Cellular Growth of CNE1 (% vs Control)
100
80
60
40
20
0
1E-8
1E-7
1E-6
1E-5
1E-4
Dose concentration (M)
1a
1f
1i
KAu$^{III}$Cl$_4$
Pt(NH$_3$)$_2$Cl$_2$

FIG. 7

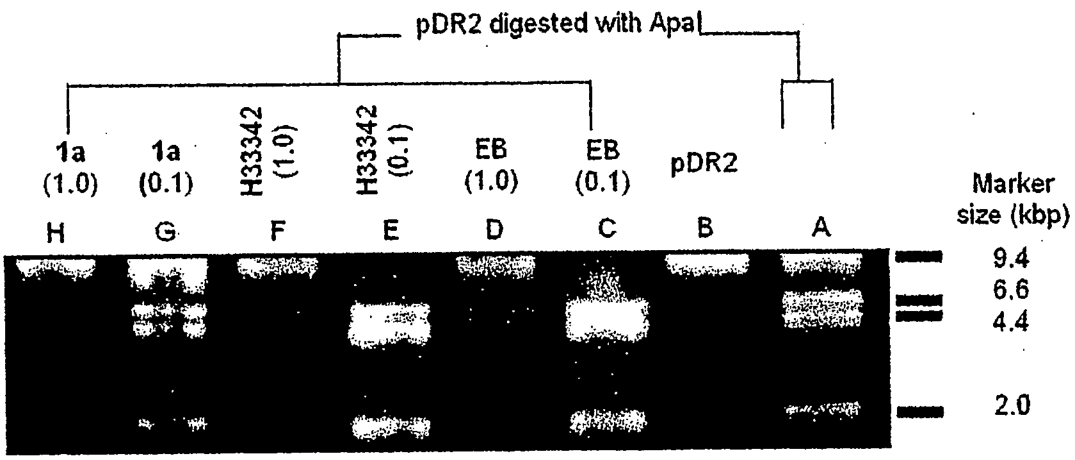
pDR2 digested with ApaI
1a (1.0)
1a (0.1)
H33342 (1.0)
H33342 (0.1)
EB (1.0)
EB (0.1)
pDR2
H
G
F
E
D
C
B
A
Marker size (kbp)
9.4
6.6
4.4
2.0

FIG. 8

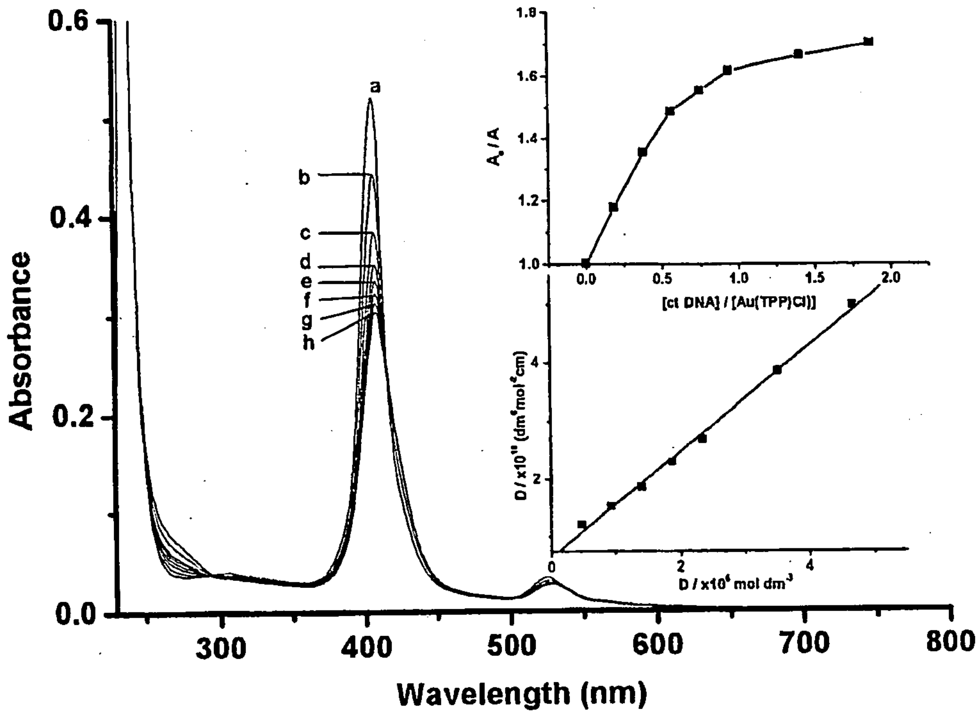

0.6
0.4
0.2
0.0
Absorbance
300
400
500
600
700
800
Wavelength (nm)
a
b
c
d
e
f
g
h
1.8
1.6
1.4
1.2
1.0
A0 / A
0.0
0.5
1.0
1.5
2.0
[ct DNA] / [Au(TPP)Cl)]
D / x10^6 mol dm^-3


FIG. 9

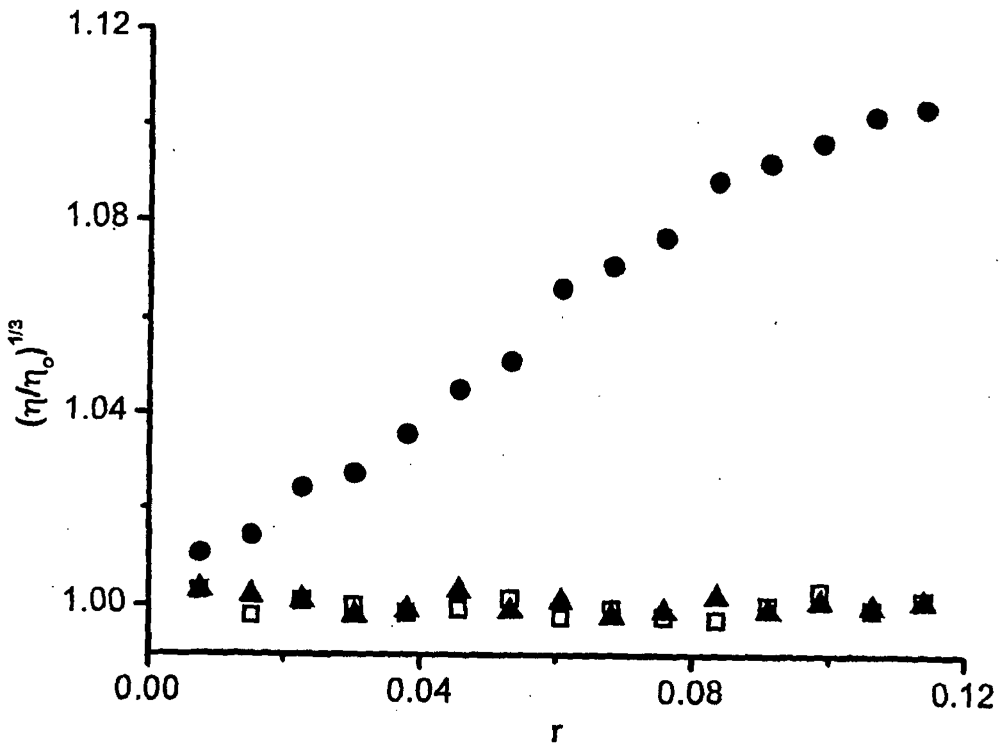


FIG. 10

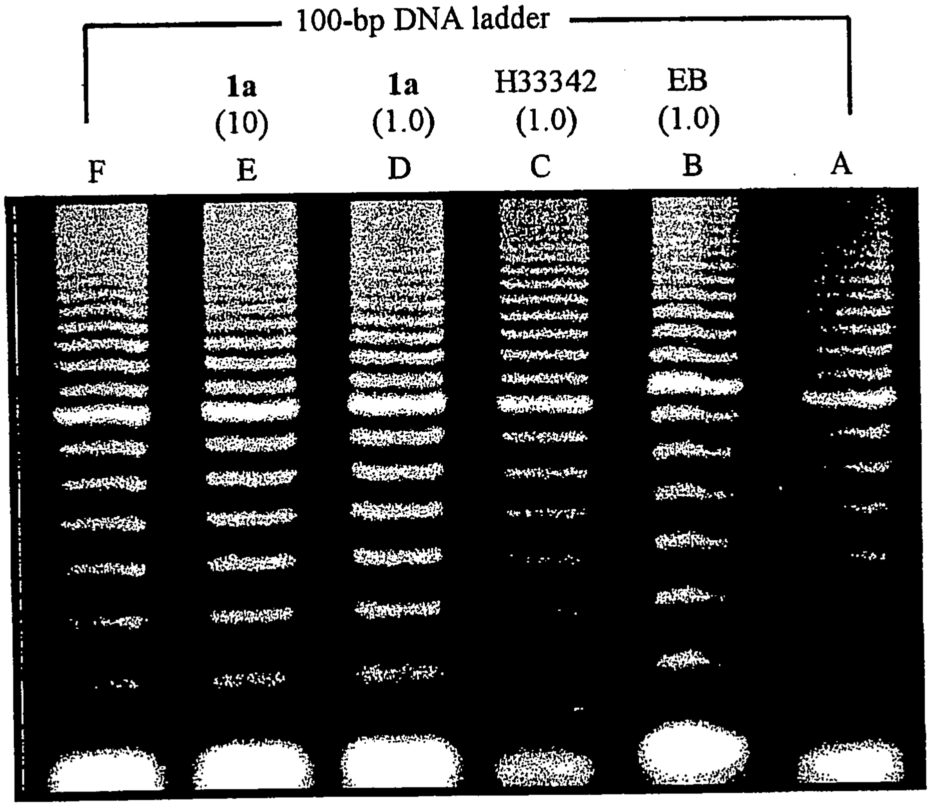


FIG. 11

(a) (b)

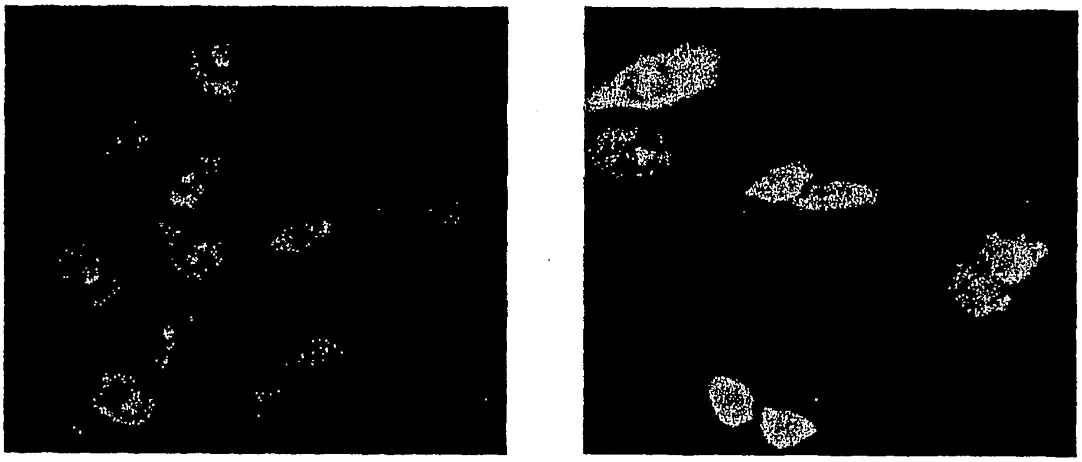

FIG. 12

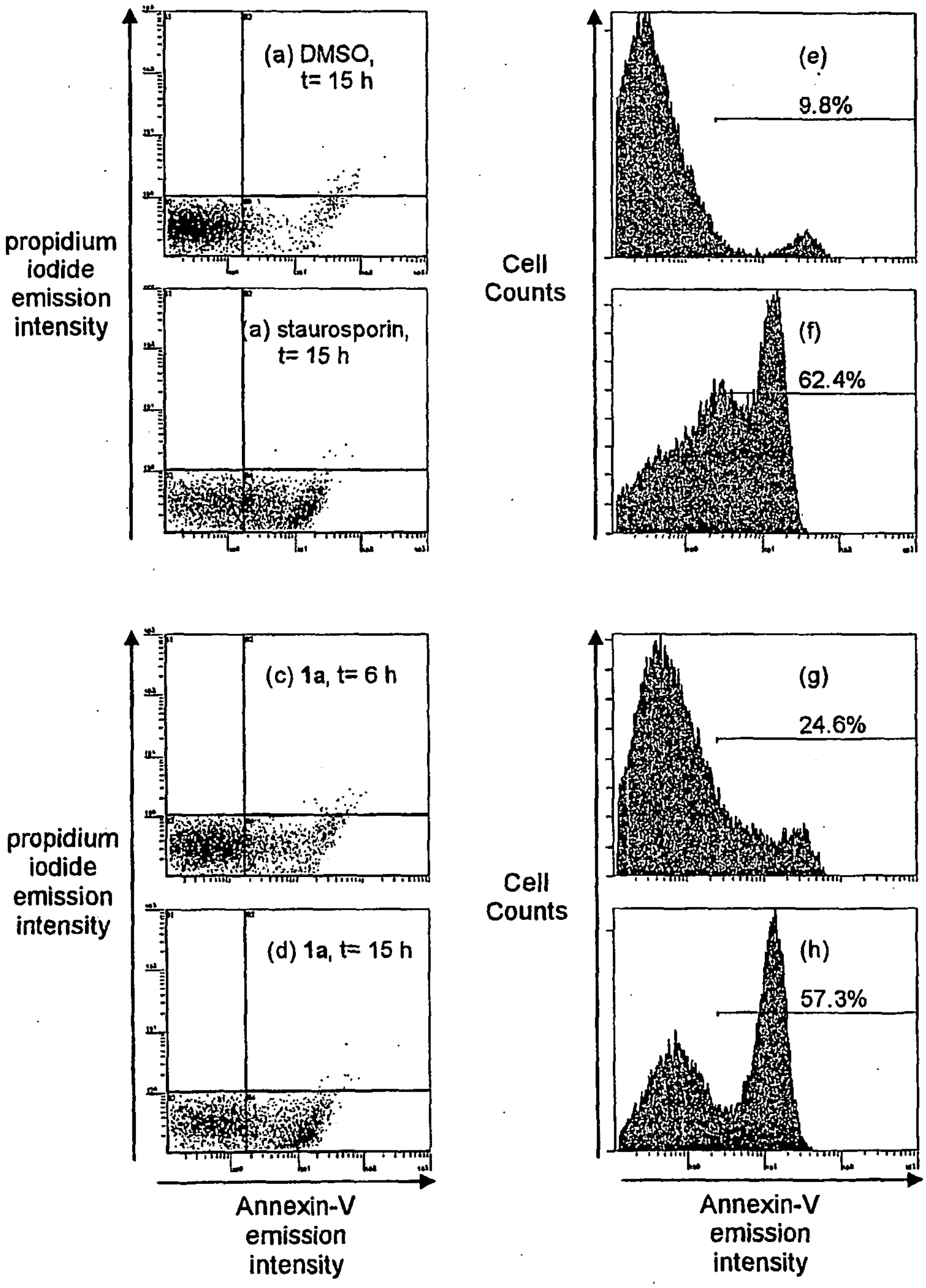
(a) DMSO, t= 15 h
(a) staurosporin, t= 15 h
(c) 1a, t= 6 h
(d) 1a, t= 15 h
propidium iodide emission intensity
propidium iodide emission intensity
Annexin-V emission intensity
(e)
9.8%
(f)
62.4%
(g)
24.6%
(h)
57.3%
Cell Counts
Cell Counts
Annexin-V emission intensity

FIG. 13

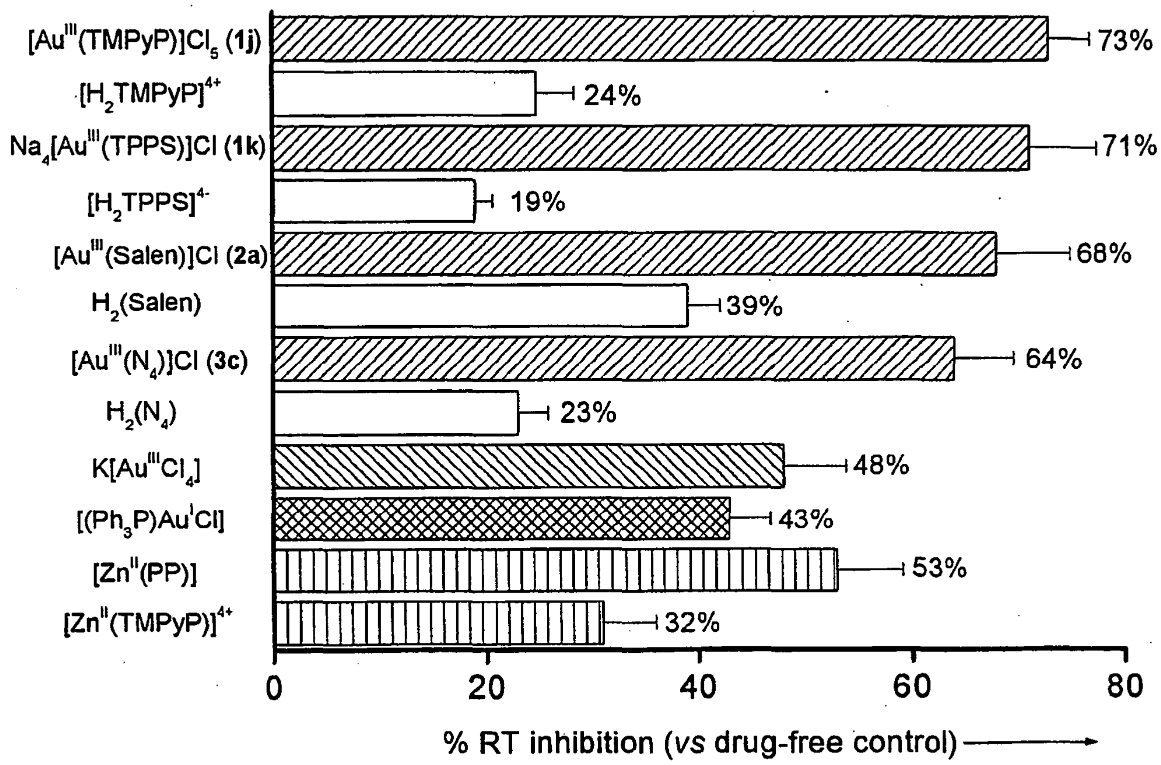
[Au^III(TMPyP)]Cl5 (1j)
73%
[H2TMPyP]4+
24%
Na4[Au^III(TPPS)]Cl (1k)
71%
[H2TPPS]4-
19%
[Au^III(Salen)]Cl (2a)
68%
H2(Salen)
39%
[Au^III(N4)]Cl (3c)
64%
H2(N4)
23%
K[Au^IIICl4]
48%
[(Ph3P)Au^ICl]
43%
[Zn^II(PP)]
53%
[Zn^II(TMPyP)]4+
32%
0
20
40
60
80
% RT inhibition (vs drug-free control)

FIG. 14

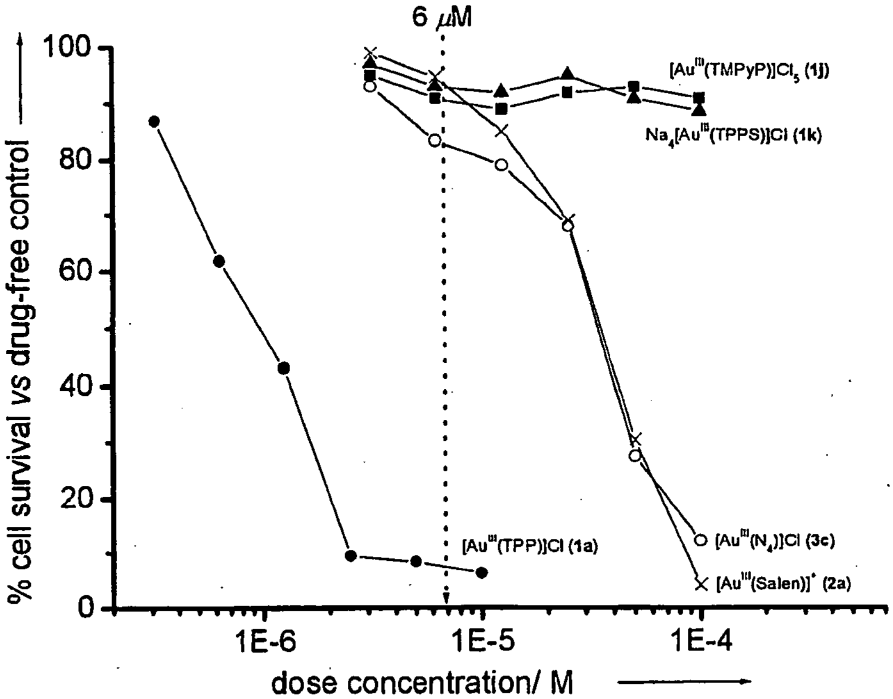
6 μM
100
80
60
40
20
0
% cell survival vs drug-free control
1E-6
1E-5
1E-4
dose concentration/ M
[Au^III(TMPyP)]Cl5 (1j)
Na4[Au^III(TPPS)]Cl (1k)
[Au^III(TPP)]Cl (1a)
[Au^III(N4)]Cl (3c)
[Au^III(Salen)]+ (2a)

FIG. 15

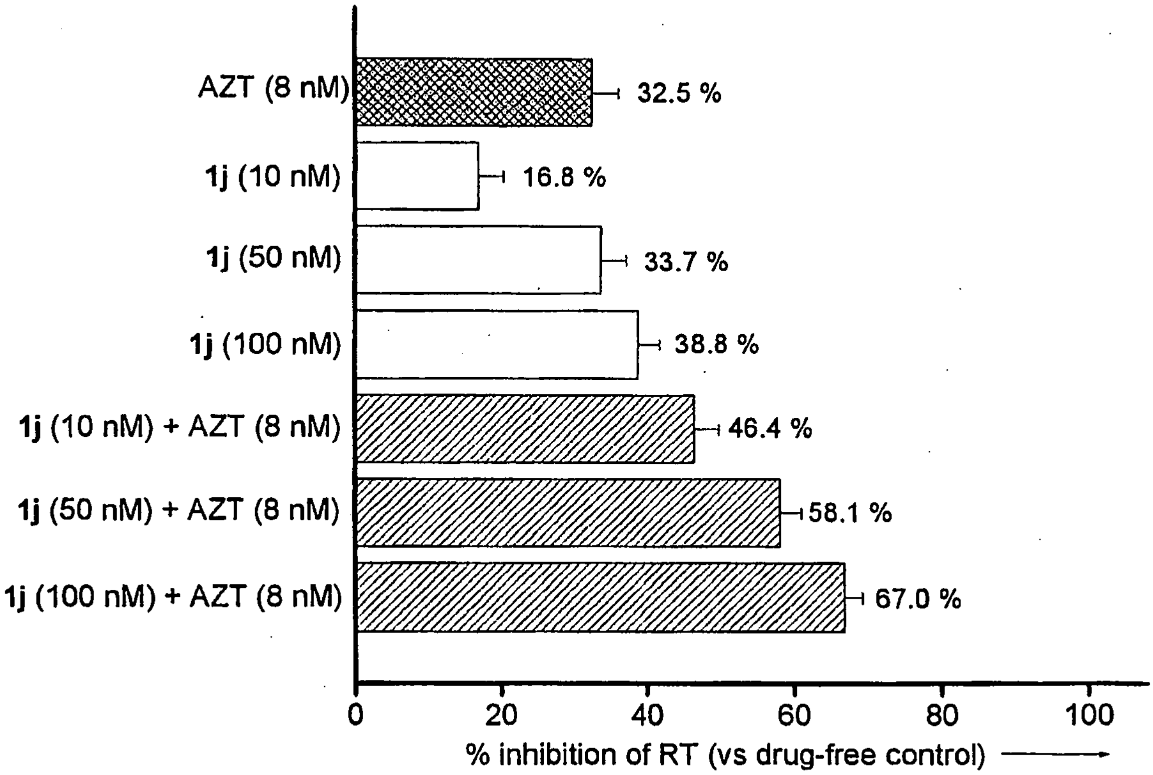
AZT (8 nM)
32.5 %
1j (10 nM)
16.8 %
1j (50 nM)
33.7 %
1j (100 nM)
38.8 %
1j (10 nM) + AZT (8 nM)
46.4 %
1j (50 nM) + AZT (8 nM)
58.1 %
1j (100 nM) + AZT (8 nM)
67.0 %
0
20
40
60
80
100
% inhibition of RT (vs drug-free control)

FIG. 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0012512 A1
- US 6087493 A

### Non-patent literature cited in the description


- *Chem. Rev.,* 1999, 99
- Topiccs in Biological Inorganic Chemistry: Metallopharmaceuticals I. **CLARKE et al.** DNA Interactions. Springer, 1999
- **SHAW, III.** Gold-Progress in Chemistry, Biochemistry and Technology. Wiley, 1999, 259
- **SIMON et al.** *Cancer,* 1965, vol. 44, 1979
- **SHAW, III.** Uses of Inorganic Chemistry in Medicine. Royal Society of Chemistry, 1999, 27
- **HOKE et al.** *Toxicol. Appl. Pharmacol.,* 1989, vol. 100, 293
- **TIEKINK.** *Critical Rev. Oncology/Hematology,* 2002, vol. 42, 225-248
- **PIEPER et al.** Topics in Biological Inorganic Chemistry: Metallopharmaceuticals I, DNA Interactions. Springer, 1999, 171-199
- **MESSORI et al.** *J. Med. Chem.,* 2000, vol. 43, 3541
- **FLEISCHER et al.** *Inorg. Nucl. Chem. Lett.,* 1969, vol. 5, 373
- **FALK, J. E.** Porphyrins and Metalloporphyrins. Elsevier, 1964
- **JAMIN et al.** *Inorganica Chimica Acta,* 1978, vol. 27, 135
- **ABOU-GAMRA et al.** *J. Chem. Soc., Faraday Trans.,* 1986, vol. 2 (82), 2337
- **ABOU-GAMRA et al.** *J. Chem. Soc., Faraday Trans. 2,* 1986, vol. 82, 2337
- **MILGROM, L. R.** The Colours of Life. Oxford University Press Inc, 1997
- **KADISH, K. M. et al.** The Porphyrin Handbook. Academic Press, 2000
- **BENNETT et al.** *Proc. Natl. Acad. Sci. USA,* 2000, vol. 97, 9476
- **GULIAEV et al.** *Biochemistry,* 1999, vol. 38, 15425
- **LIPSCOMB et al.** *Biochemistry,* 1996, vol. 35, 2818
- **MARZILLI et al.** *J. Am. Chem. Soc.,* 1992, vol. 114, 7575
- **MESTRE et al.** *Biochemisny,* 1996, vol. 35, 9140
- **HILL et al.** *Proc. Natl. Acad. Sci. USA,* 1995, vol. 92, 12126
- **HAN et al.** *J. Am. Chem. Soc.,* 1999, vol. 121, 3561-3570
- **MITSUYA et al.** *Science,* 1990, vol. 249, 1533
- Anti-AIDS Drug Development: Challenges, Strategies and Prospects. 1995, 239
- **JACOBO-MOLINA et al.** *Proc. Natl. Acad Sci. USA,* 1991, vol. 90, 6320
- **HUANG et al.** *Science,* 1998, vol. 282, 1669
- **COHEN.** *Science,* 1997, vol. 277, 32
- **PATERSON et al.** *J. Biol. Chem.,* 1999, vol. 274, 1549
- **STAUDINGER et al.** *Proc. Assoc. Am. Phys.,* 1996, vol. 108, 47
- **BARNHOLTZ et al.** *Inorg. Chem.,* 2001, vol. 40, 972
- **DAR et al.** *J. Chem. Soc., Dalton Trans.,* 1992, 1907
- **BANERJEE et al.** *Ind J. Chem.,* 1984, vol. 23A, 555
- **MURRAY et al.** *J. Organomet. Chem.,* 1973, vol. 61, 451
- **INAZU.** *Bull. Chem. Soc. Jpn.,* 1966, vol. 39, 1065
- **PASCOLO et al.** Journal of Biological Chemistry. *American Society for Biochemistry and Molecular Biology,* 2002, vol. 277 (18
- **MACCRAGH et al.** *J. Am. Chem. Soc.,* 1965, vol. 87, 2496
- **FLEISCHER et al.** *Inorg. Nucl. Chem. Lett.,* 1969, vol. 5, 373-376
- **JAMIN et al.** *Inorg. Chim. Acta,* 1978, vol. 27, 135-143
- **GIBBS et al.** *J. Inorg. Biochem.,* 1988, vol. 32, 39-65
- **PASTERNACK.** *J. Inorg. Nucl. Chem.,* 1974, vol. 36, 599
- **ADLER.** *J. Org. Chem.,* 1967, vol. 32, 476
- **KEINAN et al.** *Inorg. Chem.,* 1992, vol. 31, 5433-5438
- **BARNETT et al.** *Tetrahed. Lett.,* 1973, vol. 30, 2887-2888
- **HURLEY et al.** *Nature Reviews Cancer,* 2002, vol. 2, 188-200

- **LIU et al.** *J. Chem. Soc., Chem. Comm.,* 1995, vol. 17, 1787-1788
- **HOLLIS et al.** *J. Am. Chem. Soc.,* 1983, vol. 105, 4293-4299
- **WARD et al.** *J. Am. Chem. Soc.,* 1987, vol. 109, 3810-3811
- **SHEN et al.** *J. Biol. Chem.,* 1986, vol. 261, 7762-7770
- **GU et al.** *Chin. J. Cancer,* 1983, vol. 2, 270-272
- **OTWINOWSKI, Z. ; MINOR, W.** Processing of X-ray Diffraction Data Collected in Oscillation Mode, Methods in Enzymology. Academic Press, 1997, vol. 276, 307-326
- **SHELDRICK, G., M.** *SHELX97. Programs for Crystal Structure Analysis,* 1997
- **CALAMAI et al.** *Inorg. Chim. Acta,* 1999, vol. 285, 309-312
- **BARNHOLTZ et al.** *Inorg. Chem.,* 2001, vol. 40, 972-976
- **SUN et al.** *Eur. J. Biochem.,* 2000, vol. 267, 5450-5457
- **JIANG, X.-K.** *Acc. Chem. Res.,* 1997, vol. 30, 283-289
- **ISAACS, N. S.** Physical Organic Chemistry. Longman Scientific & Technical, 1995
- **MOSMANN et al.** *J. Immunol. Methods,* 1983, vol. 65, 55-63
- **IKEDA et al.** *J. Am. Chem. Soc.,* 1982, vol. 104, 296-297
- **LUGO-PONCE et al.** *Coord. Chem. Rev.,* 2000, vol. 208, 169-191
- **TJAHJONO et al.** *J. Inorg. Biochem.,* 2001, vol. 85, 219-228
- **HAN et al.** *J. Inorg. Biochem.,* 2002, vol. 91, 230-236
- **NEIDLE.** S. DNA structure and recognition. Oxford University Press, 1994, 74-79
- **COHEN et al.** *Biopolymers,* 1969, vol. 8, 45-55
- **CHAN et al.** *Inorg. Chem.,* 2002, vol. 41, 3161-3171
- **EBERLE et al.** *J. Virol. Methods,* 1992, vol. 40, 347-356
- **ARGYRIS et al.** *J. Biol. Chem.,* 1991, vol. 274, 1549